# EUROPEAN PATENT APPLICATION

(11) **EP 4 471 062 A1**
(43) Date of publication of application: **04.12.2024**
(21) Application number: 23746109.0
(22) Date of filing: 17.01.2023
(51) Int. Cl.: C07K 16/28, C12N 15/13, A61K 39/395, A61P 35/00

(54) **ANTI-CD73 ANTIBODY AND USE THEREOF**

(30) Priority: 25.01.2022 CN 202210098976
(71) Applicant: CSPC Megalith Biopharmaceutical Co., Ltd., Shijiazhuang, Hebei 050025 (CN)
(72) Inventor: DING, Huandi, Shijiazhuang, Hebei 050025 (CN); HUI, Xiwu, Shijiazhuang, Hebei 050025 (CN); YAO, Bing, Shijiazhuang, Hebei 050025 (CN); DAN, Mo, Shijiazhuang, Hebei 050025 (CN); JIN, Ziyi, Shijiazhuang, Hebei 050025 (CN); GUO, Qingjuan, Shijiazhuang, Hebei 050025 (CN); YUAN, Can, Shijiazhuang, Hebei 050025 (CN); LI, Wenbin, Shijiazhuang, Hebei 050025 (CN); WANG, Chao, Shijiazhuang, Hebei 050025 (CN)
(74) Representative: Gille Hrabal Partnerschaftsgesellschaft mbB Patentanwälte
(86) International application number: PCT/CN2023/072527
(87) International publication number: WO 2023/143227

(57) **Abstract**

The present application relates to a novel anti-CD73 antibody and use thereof. The anti-CD73 antibody can be used for treating tumors, particularly tumors expressing CD73 (CD73+) on the surface of tumor cells.

## Description

### CROSS-REFERENCE TO RELATED APPLICATIONS

The present application claims priority to the Chinese Patent Application No. 202210098976.1 filed on January 25, 2022, the content of which is incorporated herein by reference in its entirety and for all purposes.

### TECHNICAL FIELD

The present application relates generally to the field of biopharmaceuticals, and in particular, to a novel anti-CD73 antibody or an antigen-binding fragment thereof, a pharmaceutical composition comprising the antibody or the antigen-binding fragment thereof, and pharmaceutical use of the antibody or the antigen-binding fragment thereof.

### BACKGROUND

CD73 is an ecto-5'-nucleotidase encoded by the NT5E gene, which comprises both N-terminal and C-terminal portions and is anchored to the outer surface of the cell membrane by glycosylphosphatidylinositol (GPI) to form a homodimer. CD73 is widely expressed on the surface of endothelial cells, lymphocytes, and other cells in the human body. CD73 is highly expressed in tissues of various tumors such as gastric cancer, renal cancer, prostate cancer, breast cancer (e.g., triple negative breast cancer), non-small cell adenocarcinoma, and the like, suggesting a poor prognosis. CD73 can hydrolyze extracellular adenosine monophosphate (AMP) into adenosine. Adenosine is a powerful immunosuppressive molecule that weakens the anti-tumor immunity by inhibiting the function of protective immune cells (e.g., effector T cells, NK cells, DCs, and B cells) while maintaining the function of regulatory immune cells (e.g., Tregs, MDSCs, TAMs, and CAFs), contributing to the immune escape of tumors. In addition to inhibiting the function of immune cells, an increasing number of studies have shown that CD73 can also directly stimulate the proliferation, migration and invasion of tumor cells and tumor angiogenesis, and that inhibition of the extracellular nucleotidase CD73 can reverse adenosine-mediated immunosuppression. Studies have shown that hypoxia and radiochemotherapy lead to the upregulation of PDL1, CD73 and CD47 expression. Therefore, therapeutic strategies targeting CD73 have the potential to be applied clinically as a monotherapy or in combination therapies. Currently, there are nearly 30 anti-CD73 antibody products under development, including oleclumab from Medimmune and uliledlimab from I-Mab Biopharma. The most advanced CD73 antibody is in Phase III clinical trials, with no products on the market yet. Therefore, the development and application of antibodies against CD73 are urgently needed in the art.

### SUMMARY

In a first aspect, the present application provides an anti-CD73 antibody or an antigen-binding portion thereof, comprising a heavy chain variable region and a light chain variable region, wherein the heavy chain variable region comprises a heavy chain CDR1 (HCDR1), a heavy chain CDR2 (HCDR2), and a heavy chain CDR3 (HCDR3), and the light chain variable region comprises a light chain CDR1 (LCDR1), a light chain CDR2 (LCDR2), and a light chain CDR3 (LCDR3), wherein
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15;
wherein the sequences of the HCDRs and LCDRs are defined according to Kabat.

In a second aspect, the present application provides an anti-CD73 antibody or an antigen-binding portion thereof, the anti-CD73 antibody or an antigen-binding portion thereof comprising a heavy chain variable region and a light chain variable region, wherein
the heavy chain variable region has the amino acid sequence set forth in SEQ ID NO. 4, 18, 19, 20, 22, 24, 12, 25, 26, 28, 29 or 31, or an amino acid sequence having at least 80%, at least 85%, at least 90%, at least 95% or at least 99% identity to the amino acid sequence set forth in SEQ ID NO. 4, 18, 19, 20, 22, 24, 12, 25, 26, 28, 29 or 31; and/or
the light chain variable region has the amino acid sequence set forth in SEQ ID NO. 8, 33, 34, 16, 36, 37 or 38, or an amino acid sequence having at least 80%, at least 85%, at least 90%, at least 95% or at least 99% identity to the amino acid sequence set forth in SEQ ID NO. 8, 33, 34, 16, 36, 37 or 38.

In a third aspect, the present application provides an anti-CD73 antibody or an antigen-binding portion thereof, the anti-CD73 antibody or an antigen-binding portion thereof comprising a heavy chain variable region, wherein the heavy chain variable region comprises a heavy chain CDR1 (HCDR1), a heavy chain CDR2 (HCDR2), and a heavy chain CDR3 (HCDR3), wherein
(1) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, and the sequence of the HCDR3 is the sequence set forth in SEQ ID NO. 3 or has at least 80%, 85%, 90%, 95% or 99% identity to the sequence set forth in SEQ ID NO. 3; or
(2) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, and the sequence of the HCDR3 is the sequence set forth in SEQ ID NO. 3 or has at least 80%, 85%, 90%, 95% or 99% identity to the sequence set forth in SEQ ID NO. 3; or
(3) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, and the sequence of the HCDR3 is the sequence set forth in SEQ ID NO. 3 or has at least 80%, 85%, 90%, 95% or 99% identity to the sequence set forth in SEQ ID NO. 3; or
(4) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, and the sequence of the HCDR3 is the sequence set forth in SEQ ID NO. 3 or has at least 80%, 85%, 90%, 95% or 99% identity to the sequence set forth in SEQ ID NO. 3; or
(5) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, and the sequence of the HCDR3 is the sequence set forth in SEQ ID NO. 11 or has at least 80%, 85%, 90%, 95% or 99% identity to the sequence set forth in SEQ ID NO. 11; or
(6) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, and the sequence of the HCDR3 is the sequence set forth in SEQ ID NO. 11 or has at least 80%, 85%, 90%, 95% or 99% identity to the sequence set forth in SEQ ID NO. 11; or
(7) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, and the sequence of the HCDR3 is the sequence set forth in SEQ ID NO. 11 or has at least 80%, 85%, 90%, 95% or 99% identity to the sequence set forth in SEQ ID NO. 11;
wherein the sequences of the HCDRs are defined according to Kabat.

In a fourth aspect, the present application provides an anti-CD73 antibody or an antigen-binding portion thereof, the anti-CD73 antibody or an antigen-binding portion thereof comprising a light chain variable region, wherein the light chain variable region comprises a light chain CDR1 (LCDR1), a light chain CDR2 (LCDR2), and a light chain CDR3 (LCDR3), wherein
(1) the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
(2) the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
(3) the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
(4) the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15;
wherein the sequences of the LCDRs are defined according to Kabat.

In a fifth aspect, the present application provides an isolated nucleic acid molecule encoding the anti-CD73 antibody or the antigen-binding portion thereof according to the first to fourth aspects. In a sixth aspect, the present application provides a vector, comprising the nucleic acid molecule according to the fifth aspect.

In a seventh aspect, the present application provides a host cell, comprising the nucleic acid molecule according to the fifth aspect or the vector according to the sixth aspect.

In an eighth aspect, the present application provides an antibody-drug conjugate, comprising the anti-CD73 antibody or the antigen-binding portion thereof according to the first to fourth aspects conjugated to a therapeutic agent.

In a ninth aspect, the present application provides a pharmaceutical composition, comprising the anti-CD73 antibody or the antigen-binding portion thereof according to the first to fourth aspects or the antibody-drug conjugate according to the eighth aspect, and a pharmaceutically acceptable carrier.

In a tenth aspect, the present application provides use of the anti-CD73 antibody or the antigen-binding portion thereof according to the first to fourth aspects, the nucleic acid molecule according to the fifth aspect, the vector according to the sixth aspect, the host cell according to the seventh aspect, or the antibody-drug conjugate according to the eighth aspect for the manufacturing of a medicament for the treatment of a tumor, particularly a tumor expressing CD73 (CD73+) on the surface of tumor cells, in an individual.

In an eleventh aspect, the present application provides a method for treating a tumor, particularly a tumor expressing CD73 (CD73+) on the surface of tumor cells, in an individual, wherein the method comprises administering to the individual a therapeutically effective amount of the anti-CD73 antibody or the antigen-binding portion thereof according to the first to fourth aspects, the antibody-drug conjugate according to the eighth aspect, or the pharmaceutical composition according to the ninth aspect.

In a twelfth aspect, the present application provides a conjugate, comprising the anti-CD73 antibody or the antigen-binding portion thereof according to the first to fourth aspects and a detectable label.

In a thirteenth aspect, the present application provides a fusion protein, comprising the anti-CD73 antibody or the antigen-binding portion thereof according to the first to fourth aspects.

### BRIEF DESCRIPTION OF THE DRAWINGS

FIG. 1 shows the assay results for the binding ability of exemplary antibodies of the present application to soluble CD73.
FIG. 2 shows the assay results for the binding ability of exemplary humanized antibodies of the present application to soluble CD73.
FIG. 3 shows the assay results for the binding ability of exemplary humanized antibodies of the present application to CD73 on the surface of human breast cancer MDA-MB-231 cells.
FIG. 4 shows the assay results for the binding ability of exemplary humanized antibodies of the present application to CD73 on the surface of human ovarian cancer SKOV3 cells.
FIG. 5 shows the assay results for the binding ability of exemplary humanized antibodies of the present application to cynomolgus monkey CD73 on the surface of cells.
FIG. 6 shows the experimental results for the inhibition of enzymatic activity of CD73 protein by exemplary humanized antibodies of the present application.
FIG. 7 shows the experimental results for the inhibition of enzymatic activity of CD73 protein on the surface of cells by exemplary humanized antibodies of the present application.
FIG. 8 shows the experimental results for CD73 internalization induced by exemplary humanized antibodies of the present application.
FIG. 9A shows the test results for the recognition of the N-terminus of CD73 protein by exemplary humanized antibodies of the present application.
FIG. 9B shows the test results for the recognition of the C-terminus of CD73 protein by exemplary humanized antibodies of the present application.
FIG. 10A shows the results for the changes in graft tumor volume in A375 tumor-bearing mice resulting from administration of an exemplary antibody of the present application and other test substances.
FIG. 10B shows the results for the changes in graft tumor volume in individual A375 tumor-bearing mice resulting from administration of an exemplary antibody of the present application and other test substances.
FIG. 10C shows the results for the effects of administration of an exemplary antibody of the present application and other test substances on the body weight of A375 tumor-bearing mice.
FIG. 11A shows the results for the changes in graft tumor volume in CT26 tumor-bearing mice resulting from administration of an exemplary humanized antibody of the present application and other test substances.
FIG. 11B shows the results for the changes in graft tumor volume in individual CT26 tumor-bearing mice resulting from administration of an exemplary humanized antibody of the present application and other test substances.
FIG. 11C shows the results for the effects of administration of an exemplary humanized antibody of the present application and other test substances on the body weight of CT26 tumor-bearing mice.

### Detailed Description

### Definitions

Unless defined otherwise, all technical and scientific terms used herein have the same meaning as commonly understood by those of ordinary skill in the art. For the definitions and terminology in the art, professionals may refer to Current Protocols in Molecular Biology (Ausubel) for specific information. Abbreviations for amino acid residues refer to standard 3-letter and/or 1-letter codes used in the art to denote one of the 20 commonly used L-amino acids.

Although the numerical ranges and parameter approximations are shown in the broad scope of the present application, the numerical values shown in the specific examples are recorded as accurately as possible. However, any numerical values inherently contain certain errors necessarily resulting from the standard deviations found in their respective measurements. In addition, all ranges disclosed herein should be understood to encompass any and all subranges subsumed therein. For example, a recorded range of "1 to 10" should be considered to include any and all subranges between the minimum value of 1 and the maximum value of 10 (including the endpoints); that is, all subranges starting with a minimum value of 1 or greater, such as 1 to 6.1, and all subranges ending with a maximum value of 10 or less, such as 5.5 to 10. Additionally, any reference referred to as "incorporated herein" should be understood as being incorporated in its entirety.

The term "subject" or "individual" as used herein refers to a mammal, such as a human, but may also be another animal, such as a wild animal, a livestock animal, or a laboratory animal (e.g., orangutan, monkey, rat, mouse, rabbit, guinea pig, groundhog, squirrel, etc.).

The term "antigen" as used herein is a predetermined target to which an antibody can selectively bind. Examples of the antigen include, but are not limited to, polypeptides, sugars, nucleic acids, lipids, haptens, or other naturally occurring or synthetic compounds.

Broadly, "antibody" may refer to an immunoglobulin molecule capable of specifically binding to a target via at least one antigen recognition site located in a variable region of the immunoglobulin molecule, thus encompassing an intact antibody/full-length antibody, an antibody single chain, or any antigen-binding fragment (also referred to as an "antigen-binding portion") of an antibody. When "antibody" and "antigen-binding fragment/antigen-binding portion" are present at the same time, "antibody" may be understood as being the intact body relative to the "antigen-binding fragment/antigen-binding portion", both collectively corresponding to the broad concept of an antibody.

The term "anti-CD73 antibody" or "antibody that binds to CD73" includes antibodies that are capable of binding to CD73 with sufficient affinity such that the antibodies can be used as diagnostic and/or therapeutic agents when targeting CD73. In some embodiments, the anti-CD73 antibody binds to an unrelated, non-CD73 protein to an extent less than about 10% of the extent to which the antibody binds to CD73, as determined, for example, by fluorescence-activated cell sorting (FACS) analysis or immunoassays (e.g., radioimmunoassay (RIA)). For an antibody that "specifically binds" to CD73 or is "specific" for CD73, in certain embodiments, the antibody that binds to CD73 has a dissociation constant (KD) less than or equal to 500 nM, 100 nM, 10 nM, 9 nM, 8 nM, 7 nM, 6 nM, 5 nM, 4 nM, 0.9 nM, 0.8 nM, 0.7 nM, 0.6 nM, 0.5 nM, 0.4 nM, 0.3 nM, 0.2 nM, or 0.1 nM. In some embodiments, the anti-CD73 antibody binds to a CD73 protein epitope that is conservative between CD73 from different species (e.g., between human and cynomolgus monkey).

The term "agonist antibody" refers to an antibody that elicits a response, e.g., an antibody that mimics at least one functional activity of a polypeptide of interest. Agonist antibodies include antibodies that are mimetics of a ligand, for example, wherein the ligand binds to a cell surface receptor and this binding induces cell signal transduction or activity through an intracellular cell signal transduction pathway, and wherein the antibodies induce similar cell signal transduction or activation.

"Full-length antibody" refers to a protein comprising at least two heavy (H) chains and two light (L) chains interconnected by disulfide bonds. Each of the heavy chains comprises a heavy chain variable region (abbreviated as VH) and a heavy chain constant region. The heavy chain constant region comprises three domains,i.e., CH1, CH2, and CH3. Each of the light chains comprises a light chain variable region (abbreviated as VL) and a light chain constant region. The light chain constant region comprises one domain, i.e., CL. The VH and VL regions can be further subdivided into multiple hypervariable regions known as complementarity determining regions (CDRs), between which more conservative regions known as framework regions (FRs) are distributed. Each of VH and VL consists of three CDRs and four FRs, arranged from the amino terminus to the carboxyl terminus in the following order: FR1, CDR1, FR2, CDR2, FR3, CDR3, FR4. These variable regions of the heavy and light chains comprise binding domains that interact with antigens. The constant region of an antibody can mediate the binding of the immunoglobulin to host tissues or factors, including various cells of the immune system (e.g., effector cells) and the first component (Clq) of the classical complement system. The full-length antibody may be an antibody of any class, such as IgD, IgE, IgG, IgA, or IgM (or a subclass thereof), but the antibody does not need to belong to any particular class. Immunoglobulins can be divided into different classes depending on amino acid sequences of the antibody heavy chain constant domains. Generally, there are five main classes of immunoglobulins: IgA, IgD, IgE, IgG, and IgM, and several of these classes can be further divided into subclasses (isotypes), such as IgG1, IgG2, IgG3, IgG4, IgA1, and IgA2. The heavy chain constant domains corresponding to the different immunoglobulin classes are referred to as α, δ, ε, γ, and µ, respectively. The subunit structures and three-dimensional structures of different classes of immunoglobulins are well known. Chimeric or humanized antibodies are also encompassed by the antibodies according to the present application. It is well known to those skilled in the art that the complementarity determining regions (CDRs, generally CDR1, CDR2, and CDR3) are regions in a variable region that have the greatest impact on the affinity and specificity of an antibody. There are various common definitions for the CDR amino acid sequences in VH or VL, such as the Kabat definition, IMGT definition, Chothia definition, etc. For variable region amino acid sequences of a given antibody, the CDR amino acid sequences in the VH and VL amino acid sequences can generally be determined according to different definitions. In embodiments of the present application, the CDR amino acid sequences are defined using Kabat. For variable region amino acid sequences of a given antibody, the CDR amino acid sequences in the variable region amino acid sequence can be analyzed in a variety of ways.

The term "murine antibody" refers to an antibody that is derived from the fusion of B cells of an immunized mouse with myeloma cells, the selection of murine hybrid fusion cells capable of both infinite proliferation and antibody secretion, and then screening, preparation and purification of the resulting antibodies. Murine antibodies generally have immunogenicity and therefore require subsequent humanization.

The term "humanized antibody" means an antibody obtained by grafting CDR sequences derived from another mammalian species, such as a mouse species, onto human framework sequences. To preserve the binding affinity, some residues in the backbone (referred to as FR) segments may be modified. The humanized antibodies or fragments thereof according to the present application can be prepared by techniques known to those skilled in the art.

The term "chimeric antibody" refers to an antibody in which the variable region sequences are from one species and the constant region sequences are from another species, e.g., an antibody in which the variable region sequences are from a mouse antibody and the constant region sequences are from a human antibody. The chimeric antibodies or fragments thereof according to the present application can be prepared by using genetic recombination techniques. For example, the chimeric antibody can be produced by cloning a recombinant DNA comprising a promoter and sequences encoding variable regions of the non-human, especially murine, monoclonal antibody according to the present application, and sequences encoding constant regions of a human antibody. The chimeric antibodies of the present application encoded by such recombinant genes will be, for example, murine-human chimeras whose specificity is determined by the variable regions derived from murine DNA and whose isotypes are determined by the constant regions derived from human DNA.

The term "partially humanized antibody" refers to an antibody containing constant regions from a human and variable regions (including CDRs) from a non-human source (e.g., mouse).

The term "semi-humanized antibody" refers to a type of humanized antibody in which one antibody chain comprises a murine variable region and the other antibody chain comprises a humanized variable region, thus forming a semi-humanized antibody.

The term "monoclonal antibody" refers to an antibody obtained from a population of substantially homogeneous antibodies, i.e., the individual antibodies constituting the population are identical except that naturally occurring mutations may be present in a small number of individuals.

The terms "antigen-binding fragment", "antigen-binding portion" and "antigen-binding region" as used herein are used interchangeably, which refer to a portion of an antibody that comprises amino acid residues that interact with an antigen and confer specificity and affinity for the antigen to the binding agent, and in particular refer to antibody fragments such as Fv, Fab, F(ab')₂ or Fab', or any fragment capable of increasing the half-life by chemical modification (e.g., the addition of a poly(alkylene)glycol such as polyethylene glycol ("PEGylation") (resulting in a PEGylated fragment known as Fv-PEG, scFv-PEG, Fab-PEG, F(ab')₂-PEG or Fab'-PEG) ("PEG" represents polyethylene glycol)) or by incorporation into liposomes, wherein the fragments have CD73-binding activity. Preferably, the functional fragment will consist of or comprise a part of the sequence of the heavy or light chain variable chain of the antibody from which it is derived. The partial sequence is sufficient to retain the same binding specificity and sufficient affinity as the antibody from which it is derived, and the functional fragment will comprise at least 5 amino acids, preferably 10, 15, 25, 50 and 100 contiguous amino acids of the antibody sequence from which it is derived. Examples of the antigen-binding fragment include, but are not limited to: (1) a Fab fragment, which may be a monovalent fragment having a VL-CL chain and a VH-CH1 chain; (2) an F(ab')₂ fragment, which may be a bivalent fragment having two Fab' fragments connected by a disulfide bridge (i.e., a dimer of Fab') in the hinge region; and (3) an Fv fragment having VL and VH domains of a single arm of an antibody.

The term "single-chain fragment variable (scFv)" refers to a single polypeptide chain formed by a VH domain and a VL domain connected via a peptide linker. (scFv)₂ comprises two VH domains connected via a peptide linker and two VL domains, wherein the two VL domains are combined with the two VH domains via disulfide bridges.

The terms "Fc fragment", "Fc region", "Fc domain", "Fc portion", and similar terms refer to a portion of a heavy chain constant region of an antibody, including a hinge region, and CH2 and the CH3 fragments of the constant region. The Fc region of the anti-CD73 antibody may be engineered or modified (including effector function-related modifications) to, for example, reduce or eliminate antibody-dependent cell-mediated cytotoxicity (ADCC) and/or complement-dependent cytotoxicity (CDC), which can be achieved by introducing one or more amino acid substitutions/mutations in the Fc region of the antibody.

The term "specific binding" or "specifically bind" as used herein refers to a non-random binding reaction between two molecules, such as the binding of an antibody to an antigenic epitope.

The term "multispecific antibody" refers to molecules having binding specificity for at least 2 different antigens, wherein such molecules binding to only 2 antigens are also referred to as bispecific antibodies (BsAbs).

The term "bispecific antibody" refers to an antibody that has the ability to bind to two antigenic epitopes simultaneously. The two antigenic epitopes may be on different antigens or on the same antigen. Bispecific antibodies may have a variety of structural configurations. For example, a bispecific antibody may consist of two Fc fragments and two antigen-binding portions fused thereto, respectively (similar to a native antibody except that the two arms bind to different antigenic targets or epitopes), wherein the antigen-binding portions may be in the form of single-chain fragment variables (scFvs) or in the form of Fab fragments.

In general, for the preparation of a monoclonal antibody or a functional fragment thereof, particularly a murine monoclonal antibody or a functional fragment thereof, reference can be made to the techniques described particularly in the manual "Antibodies" (Harlow and Lane, Antibodies: A Laboratory Manual, Cold Spring Harbor Laboratory, Cold Spring Harbor NY, pp. 726, 1988) or the techniques for the preparation from hybridoma cells described by Kohler and Milstein (Nature, 256: 495-497, 1975).

The term "conservative variation" or "conservative amino acid substitution" refers to those substitutions that substantially do not affect or reduce the affinity of a protein, e.g., the affinity of an antibody for CD73. For example, a human antibody that specifically binds to CD73 may comprise up to about 1, up to about 2, up to about 5, up to about 10, or up to about 15 conservative substitutions, and specifically binds to a CD73 polypeptide. The term "conservative variation" also includes the use of a substituted amino acid in place of an unsubstituted parent amino acid, so long as the antibody specifically binds to CD73. Non-conservative substitutions are those that reduce activity or binding to CD73.

In describing some embodiments involving mutations of amino acid or nucleic acid sequences, the present application uses the designation of "XaaaY" (e.g., L234A, L235A, etc.), wherein "aaa" represents the sequence position of the amino acid or base (when a specific reference sequence is present, "aaa" represents the sequential position of the residue in that reference sequence; alternatively, the position numbering can also be in accordance with the commonly used position numbering methods in the art, such as EU numbering system, etc.), "X" represents the original amino acid or base at "aaa", and "Y" represents the amino acid or base at "aaa" after the change. As an example, when describing the L234A mutation in a human heavy chain constant region, it means that leucine (L) at position 234 is mutated to alanine (A) according to the EU numbering system for the human heavy chain constant region.

Herein, when describing "combination therapy", "combined therapy", or similar terms involving drugs A and B, the route of administration and regimen for the combination therapy with drugs A and B are not subject to specific limitations, unless there is a technical conflict or incompatibility. For example, drugs A and B may be administered separately and sequentially, administered separately and simultaneously, or formulated in the same medicament for concurrent administration. Alternatively, drugs A and B may be prepared in the same composition or in the same pharmaceutical kit during manufacture. Drugs A and B may be physically separate or combined together. The administration of drugs A and B may be temporally separate, simultaneous, or overlapping.

The term "isolated" biological components (e.g., nucleic acids, proteins (including antibodies), or organelles) have been substantially isolated or purified from other biological components (i.e., other chromosomal and extra-chromosomal DNA and RNA, proteins, and organelles) in the environment in which the isolated biological components naturally occur (e.g., cells). Nucleic acids and proteins that have been "isolated" include those purified by standard purification methods. The term also includes nucleic acids and proteins prepared by recombinant expression in host cells as well as chemically synthesized nucleic acids.

The term "derived sequence" refers to a sequence that has at least 80% (preferably 85%, 90%, 95%, 98%, or 99%) sequence identity to a related sequence and still has the same or similar function as the related sequence.

The term "pharmaceutical composition" as used herein means a combination of at least one drug and optionally a pharmaceutically acceptable carrier or auxiliary material combined together to achieve a certain objective. In certain embodiments, the pharmaceutical composition includes temporally and/or spatially separated combinations, so long as they can act together to achieve the objective of the present application. For example, the components contained in the pharmaceutical composition (e.g., the antibody, the nucleic acid molecule, the combination of nucleic acid molecules, and/or the conjugate according to the present application) may be administered to an individual as a whole or separately. When administered to an individual separately, the components contained in the pharmaceutical composition may be administered to the individual simultaneously or sequentially. Preferably, the pharmaceutically acceptable carrier is water, an aqueous buffer solution, an isotonic salt solution such as PBS (phosphate buffered saline), glucose, mannitol, dextrose, lactose, starch, magnesium stearate, cellulose, magnesium carbonate, 0.3% glycerol, hyaluronic acid, ethanol, or a polyalkylene glycol such as polypropylene glycol, triglyceride, etc. The type of the pharmaceutically acceptable carrier used depends particularly on whether the composition according to the present application is formulated for oral, nasal, intradermal, subcutaneous, intramuscular or intravenous administration. The composition according to the present application may comprise a wetting agent, an emulsifier or a buffer substance as an additive. The pharmaceutical composition or pharmaceutical formulation according to the present application may be administered by any suitable route, for example, orally, nasally, intradermally, subcutaneously, intramuscularly, or intravenously.

The term "therapeutically effective amount" or "effective amount" as used herein refers to a dose sufficient to exhibit a benefit for the individual to whom it is administered. The actual amount administered, as well as the rate and time course of administration, will depend on the condition and disease severity of the subject to be treated. The therapeutic prescription (e.g., prescribed dose, etc.) is ultimately the responsibility of the general practitioner and other physicians, and depends on their judgment, generally taking into account the disease being treated, the condition of the individual patient, the site of delivery, the method of administration, and other factors known to the physician.

The EC₅₀ value primarily refers to the concentration of a drug, antibody or toxin, etc. that achieves 50% of the maximum biological effect after a particular exposure time. In pharmacology, in addition to being useful in characterizing the activation ability of an agonist in *in-vitro* experiments, the EC₅₀ value can also be useful in indicating the plasma concentration required to achieve half the maximum biological effect *in vivo.* In some literature, EC₅₀ is also used to characterize the potency (including agonistic and antagonistic effects) of a compound at the cellular level, and the EC₅₀ value can be determined by methods such as ELISA.

In the general context, the term "fusion protein" as used herein refers to a protein composed of at least two domains, wherein the individual domains are not associated with each other in a natural state and are encoded by separate genes; the individual genes are linked together and transcribed and translated as a whole, resulting in a single protein. In the technical context of the present application, "fusion protein" comprising an antibody or an antigen-binding portion refers to a product obtained by fusing the antibody or the antigen-binding portion with another biologically active protein using genetic engineering techniques. Such antibody fusion proteins possess both the antigen-binding ability of the antibody and the unique biological properties of the biologically active protein fused with the antibody.

The terms "identity/homology/similarity" regarding an amino acid or nucleic acid sequence are defined as the percentage of identical residues in an amino acid or nucleotide sequence variant that, after sequence alignment and introduction of gaps, achieves the maximum percent identity, if desired. Methods and computer programs used for alignment are well known in the art.

The term "tumor" as used herein refers to a neoplasm or solid lesion formed by abnormal cell growth. The tumor may be benign, premalignant, or malignant.

The term "malignant tumor" as used herein refers to or describes a physiological condition in a mammal, which is typically characterized by unregulated cell growth. Exemplary malignant tumors include: carcinomas, solid tumors, melanomas, sarcomas, hematological tumors, germ cell tumors, and blastomas. More specific examples of malignant tumors include: multiple myeloma, renal cancer, lung cancer including small cell lung cancer, non-small cell lung cancer, lung adenocarcinoma and lung squamous cell carcinoma, bladder cancer, breast cancer, cervical cancer, colon cancer, gastric cancer including gastrointestinal cancer, prostate cancer, pancreatic cancer, peritoneal cancer, hepatocellular carcinoma, glioblastoma, ovarian cancer, liver cancer, urinary tract cancer, hepatoma, rectal cancer, colorectal cancer, endometrial or uterine cancer, salivary gland cancer, squamous cell carcinoma (e.g., squamous epithelial cell carcinoma), vulval cancer, thyroid cancer, anal cancer, penile cancer, melanoma and B-cell lymphoma, brain cancer, and head and neck cancer, as well as associated metastases.

The term "hematological tumor" as used herein refers to a tumor caused by uncontrolled growth and proliferation of abnormal cells; in most cases, these abnormal cells originate from the bone marrow, which is also the site where blood cells are produced. Exemplary hematological tumors include various types of leukemia, multiple myeloma, and malignant lymphoma. More specific examples of hematological tumors include: acute lymphoblastic leukemia (ALL), chronic lymphocytic leukemia (CLL), acute myeloid leukemia (AML), chronic myeloid leukemia (CML), hairy cell leukemia (HCL), T-cell prolymphocytic leukemia, large granular lymphocytic leukemia, juvenile myelomonocytic leukemia, B-cell prolymphocytic leukemia, Burkitt leukemia and adult T-cell leukemia, non-Hodgkin's lymphoma, B-cell lymphoma, small lymphocytic lymphoma, lymphoplasmacytic lymphoma, primary macroglobulinemia (Waldenström macroglobulinemia), splenic marginal zone lymphoma, plasmacytoma, extranodal marginal zone B-cell lymphoma, MALT lymphoma, nodal marginal zone B-cell lymphoma (NMZL), follicular lymphoma, mantle cell lymphoma, diffuse large B-cell lymphoma, mediastinal (thymic) large B-cell lymphoma, intravascular large B-cell lymphoma, primary effusion lymphoma, Burkitt lymphoma, B-cell chronic lymphocytic lymphoma, classical Hodgkin's lymphoma, nodular lymphocyte-predominant Hodgkin's lymphoma, adult T-cell lymphoma, extranodal NK/T-cell lymphoma, nasal type, enteropathy-associated T-cell lymphoma, hepatosplenic T-cell lymphoma, blastic NK-cell lymphoma, mycosis fungoides, Sezary syndrome, primary cutaneous CD30-positive T-cell lymphoproliferative disorder, primary cutaneous anaplastic large cell lymphoma, lymphomatoid papulosis, angioimmunoblastic T-cell lymphoma, peripheral T-cell lymphoma, not otherwise specified, and anaplastic large cell lymphoma.

The term "solid tumor" as used herein refers to a tangible mass that can be detected through clinical examination such as X-ray film, CT scan, B-mode ultrasound, or palpation. Clinically diagnosed and treated solid tumors are classified into two types: malignant and benign. Malignant solid tumors include: pediatric Hodgkin's lymphoma: lymphocyte-predominant type, nodular sclerosis type, mixed cellularity type, lymphocyte depletion type; pediatric non-Hodgkin's lymphoma: precursor lymphoblastic lymphoma, small non-cleaved cell lymphoma (Burkitt/non-Burkitt lymphoma), diffuse large B-cell lymphoma, anaplastic large cell lymphoma, etc.; pediatric renal tumors: nephroblastoma (Wilms tumor), renal clear cell carcinoma, rhabdoid tumor of kidney, renal clear cell sarcoma, renal primitive neuroectodermal tumor, etc.; pediatric neuroblastoma: neuroblastoma, ganglioneuroblastoma, ganglioneuroma; pediatric extragonadal germ cell tumors: mature teratoma, immature teratoma, endodermal sinus tumor (yolk sac tumor), seminoma, dysgerminoma, chorioepithelioma, embryonal carcinoma, etc.; osteosarcoma and chondrosarcoma; pediatric rhabdomyosarcoma: embryonal type, alveolar type, pleomorphic type, etc.; pediatric soft tissue sarcomas: fibrosarcoma, malignant fibrous histiocytoma, liposarcoma, leiomyosarcoma, angiosarcoma, lymphangiosarcoma, malignant schwannoma, alveolar soft tissue sarcoma, epithelioid sarcoma, clear cell sarcoma, malignant melanoma, synovial sarcoma, desmoplastic small round cell tumor, etc.; Ewing's sarcoma family of tumors: Ewing's sarcoma, primitive neuroectodermal tumor; pediatric liver tumors: hepatoblastoma (embryonal type, fetal type, undifferentiated type), hepatocellular carcinoma; retinoblastoma; other tumors: posterior fossa medulloblastoma, nasopharyngeal carcinoma, papillary thyroid carcinoma, thymoma, pulmonary blastoma, pancreatoblastoma, islet cell tumor, ileocecal carcinoid, mesothelioma, etc. Benign solid tumors include: lymphangioma, hemangioma, thyroglossal duct cyst, etc.

In a first aspect, the present application provides an anti-CD73 antibody or an antigen-binding portion thereof, the anti-CD73 antibody or an antigen-binding portion thereof comprising a heavy chain variable region and a light chain variable region, the heavy chain variable region comprises a heavy chain CDR1 (HCDR1), a heavy chain CDR2 (HCDR2), and a heavy chain CDR3 (HCDR3), and the light chain variable region comprises a light chain CDR1 (LCDR1), a light chain CDR2 (LCDR2), and a light chain CDR3 (LCDR3), wherein
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15;
wherein the sequences of the HCDRs and LCDRs are defined according to Kabat.

In some embodiments, the heavy chain variable region has the amino acid sequence set forth in SEQ ID NO. 4, 18, 19, 20, 22, 24, 12, 25, 26, 28, 29 or 31, or an amino acid sequence having at least 80%, at least 85%, at least 90%, at least 95% or at least 99% identity to the amino acid sequence set forth in SEQ ID NO. 4, 18, 19, 20, 22, 24, 12, 25, 26, 28, 29 or 31.

In some embodiments, the light chain variable region has the amino acid sequence set forth in SEQ ID NO. 8, 33, 34, 16, 36, 37 or 38, or an amino acid sequence having at least 80%, at least 85%, at least 90%, at least 95% or at least 99% identity to the amino acid sequence set forth in SEQ ID NO. 8, 33, 34, 16, 36, 37 or 38.

In some embodiments, the sequence of the heavy chain variable region is the sequence set forth in SEQ ID NO. 4, and the sequence of the light chain variable region is the sequence set forth in SEQ ID NO. 8; or
the sequence of the heavy chain variable region is the sequence set forth in SEQ ID NO. 18, and the sequence of the light chain variable region is the sequence set forth in SEQ ID NO. 33; or
the sequence of the heavy chain variable region is the sequence set forth in SEQ ID NO. 18, and the sequence of the light chain variable region is the sequence set forth in SEQ ID NO. 34; or
the sequence of the heavy chain variable region is the sequence set forth in SEQ ID NO. 19, and the sequence of the light chain variable region is the sequence set forth in SEQ ID NO. 33; or
the sequence of the heavy chain variable region is the sequence set forth in SEQ ID NO. 19, and the sequence of the light chain variable region is the sequence set forth in SEQ ID NO. 34; or
the sequence of the heavy chain variable region is the sequence set forth in SEQ ID NO. 20, and the sequence of the light chain variable region is the sequence set forth in SEQ ID NO. 33; or
the sequence of the heavy chain variable region is the sequence set forth in SEQ ID NO. 20, and the sequence of the light chain variable region is the sequence set forth in SEQ ID NO. 34; or
the sequence of the heavy chain variable region is the sequence set forth in SEQ ID NO. 22, and the sequence of the light chain variable region is the sequence set forth in SEQ ID NO. 33; or
the sequence of the heavy chain variable region is the sequence set forth in SEQ ID NO. 22, and the sequence of the light chain variable region is the sequence set forth in SEQ ID NO. 34; or
the sequence of the heavy chain variable region is the sequence set forth in SEQ ID NO. 24, and the sequence of the light chain variable region is the sequence set forth in SEQ ID NO. 33; or
the sequence of the heavy chain variable region is the sequence set forth in SEQ ID NO. 24, and the sequence of the light chain variable region is the sequence set forth in SEQ ID NO. 34; or
the sequence of the heavy chain variable region is the sequence set forth in SEQ ID NO. 12, and the sequence of the light chain variable region is the sequence set forth in SEQ ID NO. 16; or
the sequence of the heavy chain variable region is the sequence set forth in SEQ ID NO. 25, and the sequence of the light chain variable region is the sequence set forth in SEQ ID NO. 36; or
the sequence of the heavy chain variable region is the sequence set forth in SEQ ID NO. 25, and the sequence of the light chain variable region is the sequence set forth in SEQ ID NO. 37; or
the sequence of the heavy chain variable region is the sequence set forth in SEQ ID NO. 25, and the sequence of the light chain variable region is the sequence set forth in SEQ ID NO. 38; or
the sequence of the heavy chain variable region is the sequence set forth in SEQ ID NO. 26, and the sequence of the light chain variable region is the sequence set forth in SEQ ID NO. 36; or
the sequence of the heavy chain variable region is the sequence set forth in SEQ ID NO. 26, and the sequence of the light chain variable region is the sequence set forth in SEQ ID NO. 37; or
the sequence of the heavy chain variable region is the sequence set forth in SEQ ID NO. 26, and the sequence of the light chain variable region is the sequence set forth in SEQ ID NO. 38; or
the sequence of the heavy chain variable region is the sequence set forth in SEQ ID NO. 28, and the sequence of the light chain variable region is the sequence set forth in SEQ ID NO. 36; or
the sequence of the heavy chain variable region is the sequence set forth in SEQ ID NO. 28, and the sequence of the light chain variable region is the sequence set forth in SEQ ID NO. 37; or
the sequence of the heavy chain variable region is the sequence set forth in SEQ ID NO. 28, and the sequence of the light chain variable region is the sequence set forth in SEQ ID NO. 38; or
the sequence of the heavy chain variable region is the sequence set forth in SEQ ID NO. 29, and the sequence of the light chain variable region is the sequence set forth in SEQ ID NO. 36; or
the sequence of the heavy chain variable region is the sequence set forth in SEQ ID NO. 29, and the sequence of the light chain variable region is the sequence set forth in SEQ ID NO. 37; or
the sequence of the heavy chain variable region is the sequence set forth in SEQ ID NO. 29, and the sequence of the light chain variable region is the sequence set forth in SEQ ID NO. 38; or
the sequence of the heavy chain variable region is the sequence set forth in SEQ ID NO. 31, and the sequence of the light chain variable region is the sequence set forth in SEQ ID NO. 36; or
the sequence of the heavy chain variable region is the sequence set forth in SEQ ID NO. 31, and the sequence of the light chain variable region is the sequence set forth in SEQ ID NO. 37; or
the sequence of the heavy chain variable region is the sequence set forth in SEQ ID NO. 31, and the sequence of the light chain variable region is the sequence set forth in SEQ ID NO. 38.

In a second aspect, the present application provides an anti-CD73 antibody or an antigen-binding portion thereof, the anti-CD73 antibody or an antigen-binding portion thereof comprising a heavy chain variable region and a light chain variable region, wherein
the heavy chain variable region has the amino acid sequence set forth in SEQ ID NO. 4, 18, 19, 20, 22, 24, 12, 25, 26, 28, 29 or 31, or an amino acid sequence having at least 80%, at least 85%, at least 90%, at least 95% or at least 99% identity to the amino acid sequence set forth in SEQ ID NO. 4, 18, 19, 20, 22, 24, 12, 25, 26, 28, 29 or 31; and/or
the light chain variable region has the amino acid sequence set forth in SEQ ID NO. 8, 33, 34, 16, 36, 37 or 38, or an amino acid sequence having at least 80%, at least 85%, at least 90%, at least 95% or at least 99% identity to the amino acid sequence set forth in SEQ ID NO. 8, 33, 34, 16, 36, 37 or 38.

In a third aspect, the present application provides an anti-CD73 antibody or an antigen-binding portion thereof, the an anti-CD73 antibody or an antigen-binding portion thereof comprising a heavy chain variable region, wherein the heavy chain variable region comprises a heavy chain CDR1 (HCDR1), a heavy chain CDR2 (HCDR2), and a heavy chain CDR3 (HCDR3), wherein
(1) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, and the sequence of the HCDR3 is the sequence set forth in SEQ ID NO. 3 or has at least 80%, 85%, 90%, 95% or 99% identity to the sequence set forth in SEQ ID NO. 3; or
(2) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, and the sequence of the HCDR3 is the sequence set forth in SEQ ID NO. 3 or has at least 80%, 85%, 90%, 95% or 99% identity to the sequence set forth in SEQ ID NO. 3; or
(3) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, and the sequence of the HCDR3 is the sequence set forth in SEQ ID NO. 3 or has at least 80%, 85%, 90%, 95% or 99% identity to the sequence set forth in SEQ ID NO. 3; or
(4) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, and the sequence of the HCDR3 is the sequence set forth in SEQ ID NO. 3 or has at least 80%, 85%, 90%, 95% or 99% identity to the sequence set forth in SEQ ID NO. 3; or
(5) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, and the sequence of the HCDR3 is the sequence set forth in SEQ ID NO. 11 or has at least 80%, 85%, 90%, 95% or 99% identity to the sequence set forth in SEQ ID NO. 11; or
(6) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, and the sequence of the HCDR3 is the sequence set forth in SEQ ID NO. 11 or has at least 80%, 85%, 90%, 95% or 99% identity to the sequence set forth in SEQ ID NO. 11; or
(7) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, and the sequence of the HCDR3 is the sequence set forth in SEQ ID NO. 11 or has at least 80%, 85%, 90%, 95% or 99% identity to the sequence set forth in SEQ ID NO. 11;
wherein the sequences of the HCDRs are defined according to Kabat.

In a fourth aspect, the present application provides an anti-CD73 antibody or an antigen-binding portion thereof, the anti-CD73 antibody or an antigen-binding portion thereof comprising a light chain variable region, wherein the light chain variable region comprises a light chain CDR1 (LCDR1), a light chain CDR2 (LCDR2), and a light chain CDR3 (LCDR3), wherein
(1) the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
(2) the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
(3) the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
(4) the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15;
wherein the sequences of the LCDRs are defined according to Kabat.

In some embodiments of the first to fourth aspects, the anti-CD73 antibody or the antigen-binding portion thereof binds to human CD73. In some specific embodiments, the anti-CD73 antibody or the antigen-binding portion thereof binds to a CD73 protein epitope that is conservative between CD73 from different species (e.g., between human and cynomolgus monkey CD73).

In some embodiments of the first to fourth aspects, the anti-CD73 antibody or the antigen-binding portion thereof has ADCC activity.

In some embodiments of the first to fourth aspects, the anti-CD73 antibody or the antigen-binding portion thereof has CDC activity.

In some embodiments of the first to fourth aspects, the anti-CD73 antibody is an intact antibody, a single-chain fragment variable (scFv), an scFv-Fc, a bispecific antibody, or a multispecific antibody.

In some embodiments of the first to fourth aspects, the antigen-binding portion of the anti-CD73 antibody is a Fab, a Fab', an Fv, or an F(ab')₂.

In some embodiments of the first to fourth aspects, the anti-CD73 antibody is a murine antibody, a chimeric antibody, a humanized antibody, or a fully human antibody.

In some embodiments of the first to fourth aspects, the anti-CD73 antibody is a monoclonal antibody.

In some embodiments of the first to fourth aspects, the anti-CD73 antibody is an IgM, IgD, IgG, IgA or IgE type antibody. In some specific embodiments, the anti-CD73 antibody is an IgG antibody. In some more specific embodiments, the anti-CD73 antibody is an IgG1 antibody.

In some embodiments of the first to fourth aspects, the anti-CD73 antibody is of the IgG1, IgG2 or IgG4 isotype.

In some embodiments of the first to fourth aspects, the anti-CD73 antibody comprises a light chain constant region of the κ subtype or the λ subtype.

In some embodiments of the first to fourth aspects, the anti-CD73 antibody comprises a human IgG1 heavy chain constant region and a human κ light chain constant region.

In some embodiments of the first to fourth aspects, the anti-CD73 antibody or the antigen-binding portion thereof is for use in a medical therapy.

In some embodiments of the first to fourth aspects, the anti-CD73 antibody comprises a wild-type Fc region.

In some embodiments of the first to fourth aspects, the anti-CD73 antibody or the antigen-binding portion thereof is engineered to have enhanced ADCC or CDC activity. In some specific embodiments, the anti-CD73 antibody comprises an engineered Fc region that confers enhanced ADCC and/or CDC effects to the antibody.

In some embodiments of the first to fourth aspects, the anti-CD73 antibody or the antigen-binding portion thereof is engineered to have reduced ADCC or CDC activity. In some specific embodiments, the anti-CD73 antibody comprises an engineered Fc region that confers reduced ADCC and/or CDC effects to the antibody. For the CD73 target which the present application is directed and for *in-vivo* use of the antibodies described herein, antibodies with reduced or even eliminated ADCC or CDC activity may be beneficial because overly strong Fc effects may affect or even diminish the therapeutic efficacy of the antibodies. Methods to reduce or eliminate Fc effects are known in the art, e.g., 1) amino acid residue mutation engineering (primarily to reduce binding to relevant receptors), 2) glycosylation engineering, 3) IgG4 type antibody engineering, and the like. As a non-limiting example, the antibody has a human IgG1 heavy chain constant region and comprises mutations at one or more of positions L234, L235, P329 and P331, such as one or more of mutations L234A, L235A, P329A and P331S, wherein the residues are numbered according to the EU numbering system.

In some embodiments of the first to fourth aspects, the antibody has a heavy chain variable region set forth in SEQ ID NO. 20, a light chain variable region set forth in SEQ ID NO. 33, a heavy chain constant region set forth in SEQ ID NO. 39, and a light chain constant region set forth in SEQ ID NO. 40.

In some embodiments of the first to fourth aspects, the antibody has a heavy chain variable region set forth in SEQ ID NO. 31, a light chain variable region set forth in SEQ ID NO. 38, a heavy chain constant region set forth in SEQ ID NO. 39, and a light chain constant region set forth in SEQ ID NO. 40.

In a fifth aspect, the present application provides an isolated nucleic acid molecule encoding the anti-CD73 antibody or the antigen-binding portion thereof according to the first to fourth aspects. In some embodiments, the present application provides a combination of isolated polynucleotides, comprising a polynucleotide encoding a light chain of the antibody or the antigen-binding portion thereof of the present application and a polynucleotide encoding a heavy chain of the antibody or the antigen-binding portion thereof of the present application. In some embodiments, the polynucleotides are operably linked to regulatory sequences that can be recognized by a host cell transformed with the vector.

In a sixth aspect, the present application provides a vector (e.g., an expression vector), comprising the nucleic acid molecule or the combination of polynucleotides according to the fifth aspect.

In some embodiments, the expression vector of the present application comprises the nucleic acid molecule described herein or the combination of polynucleotides described herein, wherein the polynucleotides are operably linked to regulatory sequences that allow of expression of the polypeptides encoded by the polynucleotides in a host cell or a cell-free expression system. The selection of the expression vector depends on the selection of the host cell and can be made so as to have the desired expression and regulatory characteristics in the selected host cell.

"Expression vector" is a vector comprising one or more expression control sequences. "Expression control sequence" is a DNA sequence that controls and regulates the transcription and/or translation of another DNA sequence.

The nucleic acid in the vector may be operably linked to one or more expression control sequences. As used herein, "operably linked" means incorporation into a genetic construct such that the expression control sequence effectively controls the expression of the target coding sequence. Examples of the expression control sequence include promoters, enhancers, and transcription termination regions. A promoter is an expression control sequence consisting of a region of a DNA molecule typically located within 100 nucleotides upstream of the transcription start site (usually near the start site of RNA polymerase II). In order to place the coding sequence under the control of the promoter, the translation start site of the polypeptide translation reading frame must be located between 1 to about 50 nucleotides downstream of the promoter. Enhancers provide expression specificity in terms of time, location, and level. Unlike promoters, enhancers can function when located at different distances from the transcription site. Enhancers can also be located downstream of the transcription start site. When an RNA polymerase is capable of transcribing a coding sequence into mRNA, and the mRNA can then be translated into a protein encoded by the coding sequence, the coding sequence is "operably linked" to and "under the control" of an expression control sequence in a cell.

Suitable expression vectors include, but are not limited to, plasmids and virus vectors derived from, for example, bacteriophages, baculoviruses, tobacco mosaic viruses, herpesviruses, cytomegaloviruses, retroviruses, vaccinia viruses, adenoviruses, and adeno-associated viruses. Many vectors and expression systems are commercially available from companies such as Novagen (Madison, WI), Clontech (Palo Alto, CA), Stratagene (La Jolla, CA), and Invitrogen Life Technologies (Carlsbad, CA).

An expression vector may comprise a tag sequence. The tag sequence is typically expressed as a fusion with the encoded polypeptide. Such tags may be inserted at any position within the polypeptide, including the carboxyl or amino terminus. Examples of useful tags include, but are not limited to, Fc fragments, polyhistidine, green fluorescent protein (GFP), glutathione S-transferase (GST), c-myc, hemagglutinin, Flag^{™} tag (Kodak, New Haven, CT), maltose E binding protein, and protein A. In some embodiments, the nucleic acid molecule encoding the CD73 fusion polypeptide is present in a vector comprising a nucleic acid encoding one or more domains of an Ig heavy chain constant region, the domains corresponding, for example, to amino acid sequences of a hinge region, a CH2 region and a CH3 region of a human immunoglobulin Cγ1 chain (Fc fragments).

In a seventh aspect, the present application provides a host cell, comprising the nucleic acid molecule according to the fifth aspect or the vector according to the sixth aspect. In some embodiments, the host cell may be a prokaryotic host cell, a eukaryotic host cell, or a bacteriophage. The prokaryotic host cell may be *Escherichia coli*, *Bacillus subtilis*, *Streptomyces* or *Proteus mirabilis*, etc. The eukaryotic host cell may be a fungus such as *Pichia pastoris*, *Saccharomyces cerevisiae*, *Schizosaccharomyces* or *Trichoderma*, an insect cell such as *Spodoptera frugiperda*, a plant cell such as tobacco, or a mammalian cell such as a BHK cell, a CHO cell, a COS cell or a myeloma cell. In some embodiments, the host cell is preferably a mammalian cell, more preferably a BHK cell, a CHO cell, an NSO cell, or a COS cell.

In an eighth aspect, the present application provides an antibody-drug conjugate, also known as an ADC, comprising the anti-CD73 antibody or the antigen-binding portion thereof according to the first to fourth aspects conjugated to a therapeutic agent.

In some embodiments of the eighth aspect, the therapeutic agent is an anti-tumor drug, such as a cytotoxic drug, an immunopotentiator, or a radioisotope.

In some embodiments, the types of the cytotoxic drug include tubulin inhibitors (e.g., alkaloids), DNA topoisomerase inhibitors, DNA-damaging agents, antimetabolites, or anti-tumor antibiotics. In some embodiments, the tubulin inhibitor includes, but is not limited to, auristatin derivatives (e.g., MMAE (monomethyl auristatin E), MMAF (monomethyl auristatin F)) or maytansinoid alkaloid derivatives (e.g., DM1, DM4, ansamitocin, mertansine, or dolastatin, and derivatives thereof).

In some embodiments, the DNA topoisomerase inhibitor is a camptothecin analog or a DNA topoisomerase I inhibitor or a derivative thereof, such as DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxyacuminatine, topotecan, lurotecan, belotecan, exatecan, homosilatecan, 6, 8-dibromo-2-methyl-3-[2-(*D*-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-*β*-*D*-glucopyranosyl-12,13-dihydro-2,10-dihydroxy-6-[[2-hydroxy-1-(hydroxymethyl)ethyl]a mino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride, or *N*-[2-(dimethylamino) ethyl]-4-acridinecarboxamide.

In some embodiments, the DNA-damaging agent includes, but is not limited to, calicheamicins, duocarmycins, and anthramycin-derived PBDs (pyrrolobenzodiazepines).

In some embodiments, the antimetabolite includes, but is not limited to, methotrexate, 6-mercaptopurine, or 5-fluorouracil.

In some embodiments, the anti-tumor antibiotic includes, but is not limited to, polypeptide antibiotics (e.g., actinomycin D or bleomycin) or anthraquinone drugs (e.g., doxorubicin or mitoxantrone hydrochloride). In some embodiments, the radioisotope includes, but is not limited to, ²¹¹At, ¹³¹I, ¹²⁵I, ⁹⁰Y, ¹⁸⁶Re, ¹⁸⁸Re, ¹⁵³Sm, ²¹²Bi, ³²P, ⁶⁰Co, or ¹⁷⁷Lu.

In some embodiments, the immunopotentiator includes, but is not limited to, levamisole, pidotimod, imiquimod, isoprinosine, polyinosinic acid, or polyuridylic acid.

In some embodiments, the anti-CD73 antibody of the present application is covalently linked to a drug moiety via a linker. In some embodiments, the linker is a cleavable linker. In some embodiments, the linker may be cleaved under intracellular conditions. In one embodiment, the linker is hydrolyzable at a pH lower than 5.5. In some embodiments, the linker may be cleaved by an intracellular protease. In some embodiments, the linker is a cathepsin-cleavable linker. In some embodiments, the linker comprises a dipeptide. In some embodiments, the dipeptide is valine (Val)-citrulline (Cit). In some embodiments, the antibody is linked to the linker via a cysteine sulfhydryl group of the antibody. In one embodiment, the antibody is linked to the linker via an amino group (particularly an amino group of a glutamine residue) of the antibody. Non-limiting examples of the linker include mc-Val-Cit-pAB, mc-Val-Cit-pABC, mc-Val-Cit, NH2-(PEG)m-Val-Cit, and NH₂-(PEG)m-Val-Cit-pAB, wherein m is an integer of 1 to 8. In some embodiments, the linker is a non-cleavable linker, including, but not limited to, an acid-labile linker (e.g., a hydrazone linker), a disulfide bond-containing linker, a peptidase-sensitive linker (e.g., a peptide linker comprising amino acids, such as valine and/or citrulline, e.g., citrulline-valine or phenylalanine-lysine), a photo-labile linker, a dimethyl linker, a thioether linker, or a hydrophilic linker designed to avoid multidrug transporter-mediated resistance, and the like.

In a ninth aspect, the present application provides a pharmaceutical composition, comprising the anti-CD73 antibody or the antigen-binding portion thereof according to the first to fourth aspects or the antibody-drug conjugate according to the eighth aspect, and a pharmaceutically acceptable carrier.

In some embodiments, the pharmaceutical composition is for use in the treatment of a tumor in an individual.

In some embodiments, the tumor is a tumor expressing CD73 (CD73⁺) on the surface of tumor cells.

In some embodiments, the tumor is a tumor highly expressing CD73 (CD73⁺) on the surface of tumor cells. In some embodiments, the tumor highly expressing CD73 (CD73⁺) refers to a tumor in which at least 60% of tumor cells in the tumor cell population express CD73. In some embodiments, the tumor highly expressing CD73 (CD73⁺) refers to a tumor in which at least 70% of tumor cells in the tumor cell population express CD73. In some embodiments, the tumor highly expressing CD73 (CD73⁺) refers to a tumor in which at least 80% of tumor cells in the tumor cell population express CD73. In some embodiments, the tumor highly expressing CD73 (CD73⁺) refers to a tumor in which at least 90% of tumor cells in the tumor cell population express CD73. In some embodiments, the tumor highly expressing CD73 (CD73⁺) refers to a tumor in which at least 95% of tumor cells in the tumor cell population express CD73. In some embodiments, the tumor highly expressing CD73 (CD73⁺) refers to a tumor in which at least 98% of tumor cells in the tumor cell population express CD73. In some embodiments, the tumor highly expressing CD73 (CD73⁺) refers to a tumor in which at least 99% of tumor cells in the tumor cell population express CD73.

In some embodiments, the tumor is a solid tumor.

In some embodiments, the tumor is a hematological tumor.

In some embodiments, the tumor is a malignant tumor.

In some embodiments, the tumor is a cancer.

In some embodiments, the tumor is selected from: ovarian cancer, breast cancer, melanoma, colorectal cancer, gastric cancer, renal cancer, prostate cancer, pancreatic cancer, esophageal cancer, head and neck cancer, thyroid cancer, lung cancer, bladder cancer, cervical cancer, colon cancer, liver cancer, peritoneal cancer, hepatocellular carcinoma, glioblastoma, urinary tract cancer, rectal cancer, endometrial cancer, uterine cancer, salivary gland cancer, squamous cell carcinoma, vulval cancer, anal cancer, penile cancer, brain cancer, lymphoma (e.g., B-cell lymphoma), leukemia, and metastases of the tumors described above.

In some embodiments, the pharmaceutical composition may further comprise one or more of: a lubricant such as talc, magnesium stearate, and mineral oil; a wetting agent; an emulsifier; a suspending agent; a preservative such as benzoic acid, sorbic acid, and calcium propionate; a sweetener and/or a flavoring agent, and the like. In some embodiments, the pharmaceutical composition of the present application may be formulated in the form of a tablet, a pill, a powder, a lozenge, an elixir, a suspension, an emulsion, a solution, a syrup, a suppository, a capsule, or the like.

In some embodiments, the pharmaceutical composition of the present application may be delivered using any physiologically acceptable route of administration, including, but not limited to: oral administration, parenteral administration, nasal administration, rectal administration, intraperitoneal administration, intravascular injection, subcutaneous administration, transdermal administration, inhalation administration, and the like.

In some embodiments, the pharmaceutical composition for therapeutic use may be prepared for storage in the form of a lyophilized preparation or an aqueous solution by mixing reagents of the desired purity with pharmaceutically acceptable carriers, excipients, and the like, as appropriate. In a tenth aspect, the present application provides use of the anti-CD73 antibody or the antigen-binding portion thereof according to the first to fourth aspects, the nucleic acid molecule according to the fifth aspect, the vector according to the sixth aspect, the host cell according to the seventh aspect, or the antibody-drug conjugate according to the eighth aspect for the manufacturing of a medicament for the treatment of a tumor, particularly a tumor expressing CD73 (CD73⁺) on the surface of tumor cells, in an individual.

In some embodiments, the tumor is a tumor expressing CD73 (CD73⁺) on the surface of tumor cells.

In some embodiments, the tumor is a tumor highly expressing CD73 (CD73⁺) on the surface of tumor cells. In some embodiments, the tumor highly expressing CD73 (CD73⁺) refers to a tumor in which at least 60% of tumor cells in the tumor cell population express CD73. In some embodiments, the tumor highly expressing CD73 (CD73⁺) refers to a tumor in which at least 70% of tumor cells in the tumor cell population express CD73. In some embodiments, the tumor highly expressing CD73 (CD73⁺) refers to a tumor in which at least 80% of tumor cells in the tumor cell population express CD73. In some embodiments, the tumor highly expressing CD73 (CD73⁺) refers to a tumor in which at least 90% of tumor cells in the tumor cell population express CD73. In some embodiments, the tumor highly expressing CD73 (CD73⁺) refers to a tumor in which at least 95% of tumor cells in the tumor cell population express CD73. In some embodiments, the tumor highly expressing CD73 (CD73⁺) refers to a tumor in which at least 98% of tumor cells in the tumor cell population express CD73. In some embodiments, the tumor highly expressing CD73 (CD73⁺) refers to a tumor in which at least 99% of tumor cells in the tumor cell population express CD73.

In some embodiments, the tumor is a solid tumor.

In some embodiments, the tumor is a hematological tumor.

In some embodiments, the tumor is a malignant tumor.

In some embodiments, the tumor is a cancer.

In some embodiments, the tumor is selected from: ovarian cancer, breast cancer, melanoma, colorectal cancer, gastric cancer, renal cancer, prostate cancer, pancreatic cancer, esophageal cancer, head and neck cancer, thyroid cancer, lung cancer, bladder cancer, cervical cancer, colon cancer, liver cancer, peritoneal cancer, hepatocellular carcinoma, glioblastoma, urinary tract cancer, rectal cancer, endometrial cancer, uterine cancer, salivary gland cancer, squamous cell carcinoma, vulval cancer, anal cancer, penile cancer, brain cancer, lymphoma (e.g., B-cell lymphoma), leukemia, and metastases of the tumors described above.

In an eleventh aspect, the present application provides a method for treating a tumor, particularly a tumor expressing CD73 (CD73⁺) on the surface of tumor cells, in an individual, wherein the method comprises administering to the individual a therapeutically effective amount of the anti-CD73 antibody or the antigen-binding portion thereof according to the first to fourth aspects, the antibody-drug conjugate according to the eighth aspect, or the pharmaceutical composition according to the ninth aspect. In some embodiments of the method for treating or preventing a disease, disorder or condition, the method comprises exposing the cells to the anti-CD73 antibody *in vitro.*

In some embodiments, the tumor is a tumor expressing CD73 (CD73⁺) on the surface of tumor cells.

In some embodiments, the tumor is a tumor highly expressing CD73 (CD73⁺) on the surface of tumor cells. In some embodiments, the tumor highly expressing CD73 (CD73⁺) refers to a tumor in which at least 60% of tumor cells in the tumor cell population express CD73. In some embodiments, the tumor highly expressing CD73 (CD73⁺) refers to a tumor in which at least 70% of tumor cells in the tumor cell population express CD73. In some embodiments, the tumor highly expressing CD73 (CD73⁺) refers to a tumor in which at least 80% of tumor cells in the tumor cell population express CD73. In some embodiments, the tumor highly expressing CD73 (CD73⁺) refers to a tumor in which at least 90% of tumor cells in the tumor cell population express CD73. In some embodiments, the tumor highly expressing CD73 (CD73⁺) refers to a tumor in which at least 95% of tumor cells in the tumor cell population express CD73. In some embodiments, the tumor highly expressing CD73 (CD73⁺) refers to a tumor in which at least 98% of tumor cells in the tumor cell population express CD73. In some embodiments, the tumor highly expressing CD73 (CD73⁺) refers to a tumor in which at least 99% of tumor cells in the tumor cell population express CD73.

In some embodiments, the tumor is a solid tumor.

In some embodiments, the tumor is a hematological tumor.

In some embodiments, the tumor is a malignant tumor.

In some embodiments, the tumor is a cancer.

In some embodiments, the tumor is selected from: ovarian cancer, breast cancer, melanoma, colorectal cancer, gastric cancer, renal cancer, prostate cancer, pancreatic cancer, esophageal cancer, head and neck cancer, thyroid cancer, lung cancer, bladder cancer, cervical cancer, colon cancer, liver cancer, peritoneal cancer, hepatocellular carcinoma, glioblastoma, urinary tract cancer, rectal cancer, endometrial cancer, uterine cancer, salivary gland cancer, squamous cell carcinoma, vulval cancer, anal cancer, penile cancer, brain cancer, lymphoma (e.g., B-cell lymphoma), leukemia, and metastases of the tumors described above.

In some embodiments of the ninth to eleventh aspects, the anti-CD73 antibody or the antigen-binding portion thereof of the present application can be used alone or in combination with an additional composition in an anti-tumor therapy. For example, the anti-CD73 antibody can be co-administered with at least one additional therapeutic agent and/or adjuvant. In some embodiments, the additional compound is a therapeutic antibody other than the anti-CD73 antibody. In some embodiments, the additional therapeutic agent is a therapeutic agent that targets the cancer immune cycle (e.g., an immune checkpoint inhibitor (e.g., an anti-PD-1 antibody, an anti-PD-L1 antibody, or an anti-CTLA4 antibody), an A2AR antagonist, or a STAT-3 inhibitor), and a combination therapy using such a therapeutic agent can be used to reduce tumor-mediated immunosuppression.

In a twelfth aspect, the present application provides a conjugate, comprising the anti-CD73 antibody or the antigen-binding portion thereof according to the first to fourth aspects and a detectable label. The detectable label is, for example, a detectable fluorescent label, a chemiluminescent label, an isotopic label, or the like.

In a thirteenth aspect, the present application provides a fusion protein, comprising the anti-CD73 antibody or the antigen-binding portion thereof according to the first to fourth aspects. Examples of the antibody fusion protein include Fab fusion proteins, Fc fusion proteins, and single-chain fragment variable (scFv) fusion proteins, and the like, which are named according to the site to which an effector protein (e.g., a cytokine) is fused.

In some embodiments of the twelfth or thirteenth aspect, the conjugate can be formed by covalently binding the anti-CD73 antibody of the present application to one or more non-antibody agents via a synthetic linker. In some embodiments, the antibody of the present application is conjugated or recombinantly fused to a diagnostic agent, a detection agent, a therapeutic agent, or any other molecule. The conjugated or recombinantly fused antibody can be used, for example, to monitor or predict the onset, occurrence, progression and/or severity of a CD73-mediated disease as part of a clinical trial method, for example, to determine the efficacy of a particular therapy. Such diagnosis and detection can be achieved, for example, by coupling the antibody to a detectable label. The detectable label includes, but is not limited to, enzymes, prosthetic groups, fluorescent labels, chemiluminescent labels, isotopic labels, and the like. In some embodiments, the antibody of the present application can be conjugated or recombinantly fused to a therapeutic moiety or drug moiety that modifies a specified biological response (which may also be the antibody-drug conjugate according to the eighth aspect). The therapeutic moiety or drug moiety is not limited to a typical chemotherapeutic agent. For example, the drug moiety may be a protein, a peptide, a polypeptide, a small molecule toxin, or the like, with the desired biological activity.

It should be understood that the above detailed description is provided solely to enable those skilled in the art to understand the contents of the present application more clearly, and is not intended to be limiting in any way. Those skilled in the art are able to make various modifications and changes to the described embodiments.

### Examples

Specific embodiments of the present application will be described in detail with reference to the following examples, but those skilled in the art will understand that the following examples are merely illustrative of the present application and should not be construed as limiting the scope of the present application.

### Example 1: Preparation of Anti-Human CD73 Monoclonal Antibodies

Healthy BALB/c mice were immunized with a recombinant human CD73 protein (Arco, Cat. No. CD3-H52H7), and after the initial immunization, booster immunizations were performed 3 times at 14-day intervals. Peripheral blood serum was collected from the mice, and the mouse serum titers were detected by an enzyme-linked immunosorbent assay (ELISA). Mice with high plasma antibody titers were selected for cell fusion. 3 days before fusion, a final boost was performed via the tail vein or intraperitoneally. On the day of fusion, the spleens of the mice were taken and prepared into single-cell suspensions; SP2/0 cells and spleen cells were mixed at a ratio of 1:2, and the fusion was performed by an electrofusion method. The cells were incubated in a CO₂ incubator at 37 °C, and when the hybridoma cells grew to a certain quantity, the hybridoma supernatants were screened by ELISA method. Positive clones were selected and subcloned by a limiting dilution method for subsequent screening. Finally, the obtained monoclonal positive cells were subjected to RNA extraction, reverse transcription and PCR amplification, heavy and light chain variable region fragments of the mouse immunoglobulins were obtained, which were then sequenced. The heavy and light chain CDR region sequences and variable region amino acid sequences of the 7C3C2 and 1F2F9 clones were finally obtained as follows:
7C3C2
   Heavy chain CDR1: SYDIN (SEQ ID NO. 1)
   Heavy chain CDR2: WIYPRDGFTKYNEKFKG (SEQ ID NO. 2)
   Heavy chain CDR3: KEGYRGDWYFDV (SEQ ID NO. 3)
   Heavy chain variable region sequence 
QVQLQQSGPELVKPGASVKLSCKASGYTFTSYDINWVKQRPGQGLEWIGWIYPRDGFTK
YNEKFKGKATLTVDTSSSTAYMELHSLTSEDSAVYFCARKEGYRGDWYFDVWGTGTTVT
VSS (SEQ ID NO.4)
   Light chain CDR1: RASGNVHNYLA (SEQ ID NO. 5)
   Light chain CDR2: NAKTLAD (SEQ ID NO. 6)
   Light chain CDR3: QHFWSTPWT (SEQ ID NO. 7)
   Light chain variable region sequence 
DIQMTQSPASLSASVGETVTITCRASGNVHNYLAWYQQKQGKSPQLLVYNAKTLADGVP
SRFSGSGSGTQYSLKINSLQPEDFGSYYCQHFWSTPWTFGGGTELEIK (SEQ ID NO. 8)
1F2F9
   Heavy chain CDR1: SYWMH (SEQ ID NO. 9)
   Heavy chain CDR2: NINPSNGGTKYNEKFKS (SEQ ID NO. 10)
   Heavy chain CDR3: RDYGSPSY (SEQ ID NO. 11)
   Heavy chain variable region sequence 
QVQLQQPGTELVKPGASVKLSCKASGYTFTSYWMHWVKQRPGHGLEWIGNINPSNGGT
KYNEKFKSKATLTVDKSSSTAYLQLSSLTSEDSAVYYCARRDYGSPSYWGQGTLVTVSA
(SEQ ID NO. 12)
   Light chain CDR1: KASQDINSYLT (SEQ ID NO. 13)
   Light chain CDR2: RTNILLD (SEQ ID NO. 14)
   Light chain CDR3: LQYHEFPVT (SEQ ID NO. 15)
   Light chain variable region sequence 
DIKMTQSPSSIYASLGERVTITCKASQDINSYLTWFQQKVGKSPKTLIYRTNILLDGVPSRF
SGSGSGQDYSLTISSLDYEDMGIYHCLQYHEFPVTFGAGTKLELK (SEQ ID NO. 16)
   Note: In the antibody variable region sequences described above, CDR sequences are underlined (determined and annotated according to the Kabat numbering system).

### Example 2: Assay on Affinity of Chimeric Antibodies by ELISA Method

According to the variable region sequence information of the monoclonal antibody encoding genes described above, a human-mouse chimeric antibody expression vector was constructed. The VH and VL were separately constructed into eukaryotic expression vectors containing the hIgG1-kappa constant region with the L234A, L235A, P329A and P331S mutations (abbreviated as hIgG1 in the present application, with the heavy chain constant region sequence set forth in SEQ ID NO. 39 and the light chain constant region sequence set forth in SEQ ID NO. 40; the subsequent examples are consistent with this).

ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPEAAGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALAASIEKTISKAKGQPREPQVYTLPPSRDE
LTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO. 39)


 (SEQ ID NO. 40)

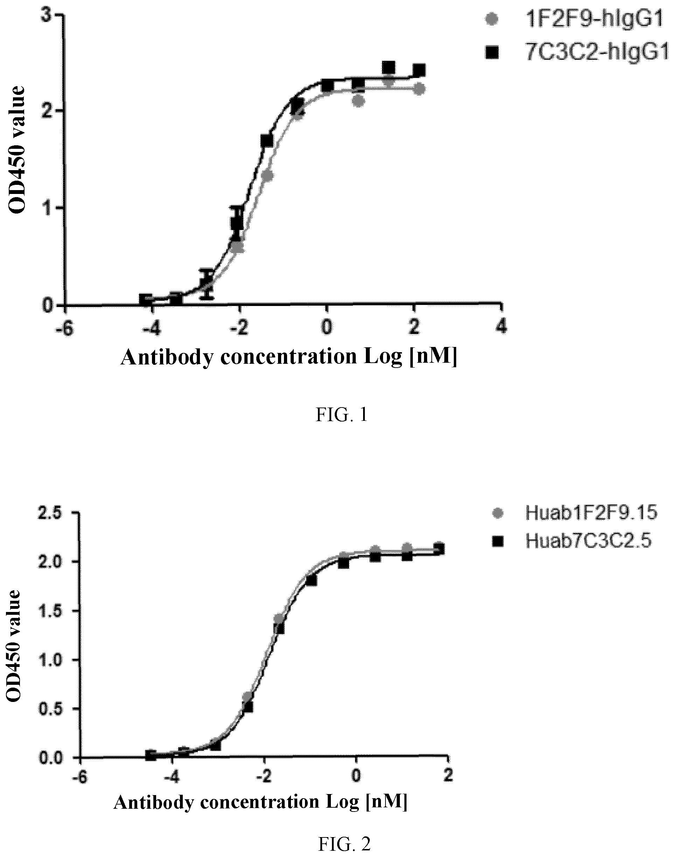

The obtained eukaryotic expression vectors were transiently transferred into HEK293 cells, and the cells were then cultured for 6 days. The culture supernatants were harvested and purified by a ProteinA column to obtain the target antibodies, which were named 7C3C2-hIgG1 and 1F2F9-hIgG1, respectively. The affinity of the antibodies for the recombinant human CD73 protein (as described in Example 1) was determined using ELISA method. A elisa plate was coated with the recombinant human CD73 protein at a concentration of 1 µg/mL and washed 3 times with a PBST buffer. Then, the plate was blocked with a PBS buffer containing 2% BSA at 37 °C for 2 h and washed 3 times with a PBST buffer. 5-fold gradient dilutions (0-133.3 nM) of the test antibodies were added, and the mixture was incubated at 37 °C for 2 h. The plate was further washed 3 times with a PBST buffer. A horseradish peroxidase-labeled secondary antibody was added, and the mixture was incubated at 37 °C for 1 h. The plate was then washed 3 times with a PBST buffer. Chromogenic reaction was performed, and the absorbance values were read after the reaction was terminated. The obtained affinity results are shown in FIG. 1 and Table 1.

**Table 1. EC₅₀ values for affinity of chimeric antibodies**

| Name of antibody | EC₅₀ value (nM) |
|---|---|
| 7C3C2-hIgG1 | 0.017 |
| 1F2F9-hIgG1 | 0.028 |

EC₅₀ values of 7C3C2-hIgG1 and 1F2F9-hIgG1 bound to the CD73 protein was 0.017 nM and 0.028 nM, respectively. Based on the data, it can be seen that the EC₅₀ values of the two antibodies met the requirements for further experiments.

### Example 3: Antibody Humanization

Homology Modeling was performed using Discovery Studio and Schrödinger Antibody Modeling. Through structural modeling and rational design, the humanized framework regions closest to the murine antibody framework regions were obtained by analysis. The humanized light chain template for the murine 7C3C2 was IGKV1-NL1; for the heavy chain, the closest human matching sequence was the IGHV1-8 gene. The humanized light chain template for the murine 1F2F9 was the IGKV1-16 gene, and the closest human heavy chain matching sequence is the IGHV1-3 gene. The CDRs of the light and heavy chains were grafted to the framework sequences of the matching light and heavy chain genes, respectively, so as to obtain humanized antibodies Hab7C3C2 and Hab1F2F9. Then, 3D models were constructed through Discovery Studio and Schrödinger Antibody Modeling to analyze the presence of any framework positions where the substitution of mouse amino acids with human amino acids affected the binding and/or CDR conformation. The sequences with back mutations are shown in Table 2 and Table 3. The relevant humanized antibodies are shown in Table 4 and Table 5.

**Table 2, Sequences with VH back mutations (CDR sequences were defined according to Kabat)**

| Construct | Mutant |
|---|---|
| Hab7C3C2-VH.1 | Heavy chain CDR1: SYDIN (SEQ ID NO. 1) |
| | Heavy chain CDR2: WIYPRDGFTGYAQKFQG (SEQ ID NO. 17) |
| | Heavy chain CDR3: KEGYRGDWYFDV (SEQ ID NO. 3) |
| | QVQLVQSGAEVKKPGASVKVSCKASGYTFTSYDINWVRQATGQGLEWMGWIYPRDGFTGYAQKFQGRVTITRNTSISTAYMELSSLRSEDTAVYYCAR KEGYRGDWYFDVWGQGTTVTVSS (SEQ ID NO. 18) |
| Hab7C3C2-VH.1a | Heavy chain CDR1: SYDIN (SEQ ID NO. 1) |
| | Heavy chain CDR2: WIYPRDGFTKYNEKFKG (SEQ ID NO. 2) |
| | Heavy chain CDR3: KEGYRGDWYFDV (SEQ ID NO. 3) |
| | QVQLVQSGAEVKKPGASVKLSCKASGYTFTSYDINWVRQATGQGLEWIGWIYPRDGFTKYNEKFKGRATLTVDTSISTAYMELSSLRSEDTAVYYCARKE GYRGDWYFDVWGQGTTVTVSS (SEQ ID NO. 19) |
| Hab7C3C2-VH.1b | Heavy chain CDR1: SYDIN (SEQ ID NO. 1) |
| | Heavy chain CDR2: WIYPRDGFTKYNEKFKG (SEQ ID NO. 2) |
| | Heavy chain CDR3: KEGYRGDWYFDV (SEQ ID NO. 3) |
| | QVQLVQSGAEVKKPGASVKVSCKASGYTFTSYDINWVRQATGQGLEWIGWIYPRDGFTKYNEKFKGRVTLTVNTSISTAYMELSSLRSEDTAVYYCARKE GYRGDWYFDVWGQGTTVTVSS (SEQ ID NO. 20) |
| Hab7C3C2-VH.1c | Heavy chain CDR1: SYDIN (SEQ ID NO. 1) |
| | Heavy chain CDR2: WIYPRDGFTKYNEKFQG (SEQ ID NO. 21) |
| | Heavy chain CDR3: KEGYRGDWYFDV (SEQ ID NO. 3) |
| | QVQLVQSGAEVKKPGASVKVSCKASGYTFTSYDINWVRQATGQGLEWIGWIYPRDGFTKYNEKFQGRVTITVNTSISTAYMELSSLRSEDTAVYYCARKE GYRGDWYFDVWGQGTTVTVSS (SEQ ID NO. 22) |
| Hab7C3C2-VH.1d | Heavy chain CDR1: SYDIN (SEQ ID NO. 1) |
| | Heavy chain CDR2: WIYPRDGFTKYAQKFQG (SEQ ID NO. 23) |
| | Heavy chain CDR3: KEGYRGDWYFDV (SEQ ID NO. 3) |
| | QVQLVQSGAEVKKPGASVKVSCKASGYTFTSYDINWVRQATGQGLEWMGWIYPRDGFTKYAQKFQGRVTITVNTSISTAYMELSSLRSEDTAVYYCAR KEGYRGDWYFDVWGQGTTVTVSS (SEQ ID NO. 24) |
| Hab1F2F9-VH.1a | Heavy chain CDR1: SYWMH (SEQ ID NO. 9) |
| | Heavy chain CDR2: NINPSNGGTKYNEKFKS (SEQ ID NO. 10) |
| | Heavy chain CDR3: RDYGSPSY (SEQ ID NO. 11) |
| | QVQLVQSGAEVKKPGASVKLSCKASGYTFTSYWMHWVRQAPGQRLEWIGNINPSNGGTKYNEKFKSRATLTVDKSASTAYLELSSLRSEDTAVYYCARR DYGSPSYWGQGTTVTVSS (SEQ ID NO. 25) |
| Hab1F2F9-VH.1b | Heavy chain CDR1: SYWMH (SEQ ID NO. 9) |
| | Heavy chain CDR2: NINPSNGGTKYNEKFKS (SEQ ID NO. 10) |
| | Heavy chain CDR3: RDYGSPSY (SEQ ID NO. 11) |
| | QVQLVQSGAEVKKPGASVKVSCKASGYTFTSYWMHWVRQAPGQRLEWIGNINPSNGGTKYNEKFKSRVTITVDKSASTAYLELSSLRSEDTAVYYCARR DYGSPSYWGQGTTVTVSS (SEQ ID NO. 26) |
| Hab1F2F9-VH.1c | Heavy chain CDR1: SYWMH (SEQ ID NO. 9) |
| | Heavy chain CDR2: NINPSNGGTKYNEKFQG (SEQ ID NO. 27) |
| | Heavy chain CDR3: RDYGSPSY (SEQ ID NO. 11) |
| | QVQLVQSGAEVKKPGASVKVSCKASGYTFTSYWMHWVRQAPGQRLEWIGNINPSNGGTKYNEKFQGRVTITVDTSASTAYLELSSLRSEDTAVYYCAR RDYGSPSYWGQGTTVTVSS (SEQ ID NO. 28) |
| Hab1F2F9-VH.1d | Heavy chain CDR1: SYWMH (SEQ ID NO. 9) |
| | Heavy chain CDR2: NINPSNGGTKYNEKFQG (SEQ ID NO. 27) |
| | Heavy chain CDR3: RDYGSPSY (SEQ ID NO. 11) |
| | QVQLVQSGAEVKKPGASVKVSCKASGYTFTSYWMHWVRQAPGQRLEWMGNINPSNGGTKYNEKFQGRVTITVDTSASTAYMELSSLRSEDTAVYYC ARRDYGSPSYWGQGTTVTVSS (SEQ ID NO. 29) |
| Hab1F2F9-VH.1e | Heavy chain CDR1: SYWMH (SEQ ID NO. 9) |
| | Heavy chain CDR2: NINPSNGGTKYSQKFQG (SEQ ID NO. 30) |
| | Heavy chain CDR3: RDYGSPSY (SEQ ID NO. 11) |
| | QVQLVQSGAEVKKPGASVKVSCKASGYTFTSYWMHWVRQAPGQRLEWMGNINPSNGGTKYSQKFQGRVTITVDTSASTAYMELSSLRSEDTAVYYC ARRDYGSPSYWGQGTTVTVSS (SEQ ID NO. 31) |

**Table 3. Sequences with VL back mutations (CDR sequences were defined according to Kabat)**

| Construct | Mutant |
|---|---|
| Hab7C3C2-VL.1 | Light chain CDR1: RASGNVHNYLA (SEQ ID NO. 5) |
| | Light chain CDR2: NAKTLAS (SEQ ID NO. 32) |
| | Light chain CDR3: QHFWSTPWT (SEQ ID NO. 7) |
| | DIQMTQSPSSLSASVGDRVTITCRASGNVHNYLAWYQQKPGKAPKLLLYNAKTLASGVPSRFSGSGSGTDYTLTISSLQPEDFATYYCQHFWSTPWTFGGG TELEIK (SEQ ID NO. 33) |
| Hab7C3C2-VL.1a | Light chain CDR1: RASGNVHNYLA (SEQ ID NO. 5) |
| | Light chain CDR2: NAKTLAD (SEQ ID NO. 6) |
| | Light chain CDR3: QHFWSTPWT (SEQ ID NO. 7) |
| | DIQMTQSPSSLSASVGDRVTITCRASGNVHNYLAWYQQKPGKAPKLLVYNAKTLADGVPSRFSGSGSGTDYTLTISSLQPEDFATYYCQHFWSTPWTFGGG TELEIK (SEQ ID NO. 34) |
| Hab1F2F9-VL.1 | Light chain CDR1: RASQDINSYLT (SEQ ID NO. 35) |
| | Light chain CDR2: RTNILLD (SEQ ID NO. 14) |
| | Light chain CDR3: LQYHEFPVT (SEQ ID NO. 15) |
| | DIQMTQSPSSLSASVGDRVTITCRASQDINSYLTWFQQKPGKAPKSLIYRTNILLDGVPSRFSGSGSGTDFTLTISSLQPEDFATYYCLQYHEFPVTFGGGTKLEI K (SEQ ID NO. 36) |
| Hab1F2F9-VL.1a | Light chain CDR1: KASQDINSYLT (SEQ ID NO. 13) |
| | Light chain CDR2: RTNILLD (SEQ ID NO. 14) |
| | Light chain CDR3: LQYHEFPVT (SEQ ID NO. 15) |
| | DIKMTQSPSSLSASVGDRVTITCKASQDINSYLTWFQQKVGKAPKTLIYRTNILLDGVPSRFSGSGSGTDYTLTISSLQPEDFATYHCLQYHEFPVTFGGGTKLEI K (SEQ ID NO. 37) |
| Hab1F2F9-VL.1b | Light chain CDR1: KASQDINSYLT (SEQ ID NO. 13) |
| | Light chain CDR2: RTNILLD (SEQ ID NO. 14) |
| | Light chain CDR3: LQYHEFPVT (SEQ ID NO. 15) |
| | DIQMTQSPSSLSASVGDRVTITCKASQDINSYLTWFQQKPGKAPKSLIYRTNILLDGVPSRFSGSGSGTDYTLTISSLQPEDFATYYCLQYHEFPVTFGGGTKLEI K (SEQ ID NO. 38) |

**Table 4: Schematic representation for 7C3C2 humanized antibody combinations**

| | Hab7C3C2-VL.1 | Hab7C3C2-VL.1a |
|---|---|---|
| Hab7C3C2-VH.1 | Hab7C3C2.1 | Hab7C3C2.2 |
| Hab7C3C2-VH.1a | Hab7C3C2.3 | Hab7C3C2.4 |
| Hab7C3C2-VH.1b | Hab7C3C2.5 | Hab7C3C2.6 |
| Hab7C3C2-VH.1c | Hab7C3C2.7 | Hab7C3C2.8 |
| Hab7C3C2-VH.1d | Hab7C3C2.9 | Hab7C3C2.10 |

**Table 5: Schematic representation for 1F2F9 humanized antibody combinations**

| | Hab1F2F9-VL.1 | Hab1F2F9-VL.1a | Hab1F2F9-VL.1b |
|---|---|---|---|
| Hab1F2F9-VH.1a | Hab1F2F9.1 | Hab1F2F9.2 | Hab1F2F9.3 |
| Hab1F2F9-VH.1b | Hab1F2F9.4 | Hab1F2F9.5 | Hab1F2F9.6 |
| Hab1F2F9-VH.1c | Hab1F2F9.7 | Hab1F2F9.8 | Hab1F2F9.9 |
| Hab1F2F9-VH.1d | Hab1F2F9.10 | Hab1F2F9.11 | Hab1F2F9.12 |
| Hab1F2F9-VH.1e | Hab1F2F9.13 | Hab1F2F9.14 | Hab1F2F9.15 |

### Example 4: Assay on Affinity of Humanized Anti-CD73 Antibodies

### 4.1. Assay on the affinity of humanized antibodies to human CD73 protein by Fortebio

The humanized antibody VH and VL were separately constructed into eukaryotic expression vectors containing the hIgG1-kappa constant region with L234A, L235A, P329A and P331S mutations (abbreviated as hIgG1 in the present application). The affinity of all humanized antibodies was assayed in this example. Human CD73 protein was immobilized onto HIS1K biosensors to achieve a binding response threshold of 0.3 nm. After a 120-second baseline step, the sensors were separately immersed in anti-CD73 antibody analytes diluted in a gradient with a 0.02% PBST buffer (each antibody was diluted in a 2-fold gradient with an initial concentration of 25 nM, resulting in 7 concentration gradients). The times for the ligand-analyte binding and dissociation were 120 s and 300 s, respectively. The data of the antigen-antibody affinity were calculated by regression according to the binding curves using an analysis software. Among all the tested antibodies, Hab7C3C2.5, Huab7C3C.9, Huab1F2F9.12, and Huab1F2F9.15 showed the highest affinity and fewer back mutations, and the results are shown in Table 6.

**Table 6: Results for the affinity of humanized antibodies to human CD73 protein assayed by Fortebio method**

| Test substance | Ka (1/Ms) | Kd (1/s) | KD (M) |
|---|---|---|---|
| Hab7C3C2.5 | 3.56E06 | 2.72E-03 | 7.653E-10 |
| Hab7C3C2.9 | 3.7E06 | 3.81E-03 | 1.03E-09 |
| Hab1F2F9.12 | 1.85E06 | 1.33E-03 | 7.195E-10 |
| Hab1F2F9.15 | 2.06E06 | 1.6E-03 | 7.787E-10 |

### 4.2. Assay on the affinity of humanized antibodies to human CD73 protein by ELISA

The affinity of the humanized antibodies to the recombinant CD73 protein was determined using an ELISA method. A elisa plate was coated with the CD73 protein at a concentration of 1 µg/mL. 5-fold gradient dilutions (0-66.7 nM) of the test antibodies were added. The obtained antibody affinity results are shown in FIG. 2 and Table 7.

**Table 7. EC₅₀ values for affinity of humanized antibodies determined by ELISA method**

| Test substance | EC₅₀ value (nM) |
|---|---|
| Hab7C3C2.5 | 0.013 |
| Hab1F2F9.15 | 0.011 |

Hab7C3C2.5 and Huab1F2F9.15 were bound to the CD73 protein with EC₅₀ values of 0.013 nM and 0.011 nM, respectively. Based on the data, it can be seen that the affinity of the humanized antibodies was not affected as compared to the murine antibodies.

### Example 5: Binding of Humanized Antibodies to Human Tumor Cells

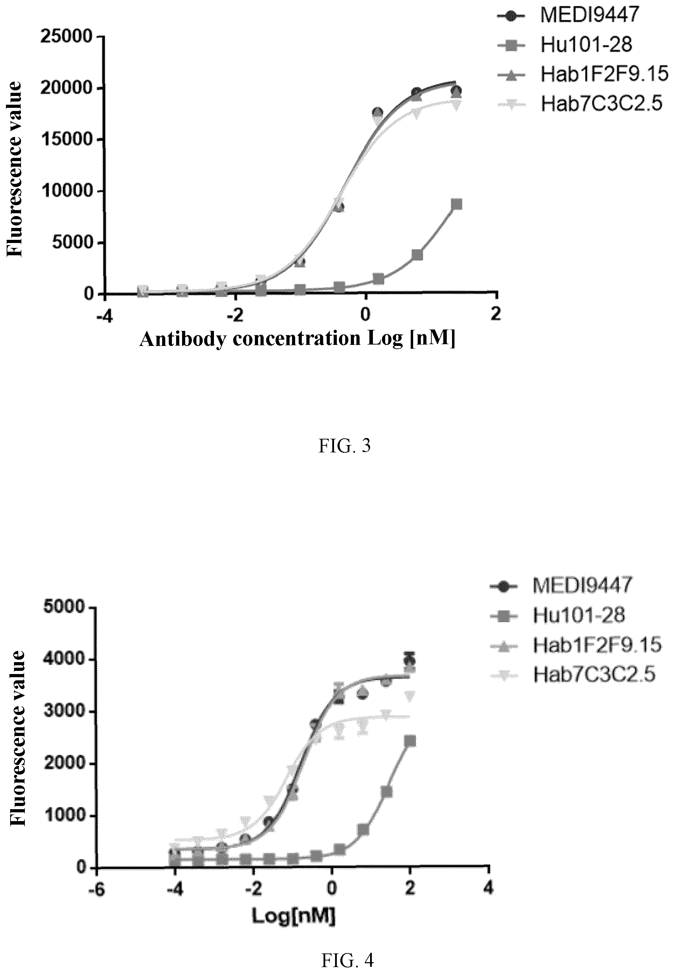

In this example, the exemplary antibodies of the present application were compared with anti-CD73 antibodies described in other patent applications. Antibodies MEDI9447 and Hu101-28 (with CDR sequences from PCT patent applications WO 2016/075099 and WO 2018/137598, respectively) were cloned into eukaryotic expression vectors containing the hIgG I-kappa constant region. The binding ability of the humanized antibodies Hab7C3C2.5 and Hab1F2F9.15 to cell surface CD73 was assessed using tumor cell lines MDA-MB-231 and SKOV3 which endogenously express human CD73 on the cell surface. The cells were seeded in a 96-well plate at a density of 2 × 10⁶ cells/mL, with 100 µL per well. 4-fold gradient dilutions (0-100 nM) of the antibodies were added, and the mixture was incubated at 4 °C for 1 h. A goat anti-human secondary antibody was added, and the mixture was incubated at 4 °C in the dark for 50 min for labeling. The fluorescence intensity of the cells was then measured by a flow cytometer. The experimental results are shown in FIGs. 3 and 4 and Table 8. The results indicate that the exemplary humanized antibodies of the present application could bind to the CD73 protein expressed on the cell lines in a dose-dependent manner; meanwhile, in terms of the ability to recognize cell surface CD73, Hab7C3C2.5 and Hab1F2F9.15 were superior to the control molecule Hu101-28 and were comparable to MEDI9447.

**Table 8. Experimental results for binding of exemplary humanized antibodies of the present application to CD73 on the surface of human tumor cells**

| Test substance | Affinity EC₅₀ at cellular level (nM) | |
|---|---|---|
| | MDA-MB-231 cells | SK-OV-3 cells |
| Hab7C3C2.5 | 0.41 | 0.07 |
| Hab1F2F9.15 | 0.48 | 0.19 |
| MEDI9447 | 0.49 | 0.17 |

### Example 6: Cross-Reactivity of Humanized Anti-CD73 Antibodies with Cynomolgus Monkey CD73

### 6.1. Assay on cross-recognition of humanized antibodies with monkey CD73 protein by Fortebio method

In this example, the cross-binding ability of the exemplary humanized anti-CD73 antibodies of the present application to cynomolgus monkey CD73 was assayed by a Fortebio experiment. The specific implementation steps included immobilizing the cynomolgus monkey CD73 protein onto HIS1K biosensors. After a 120-second baseline step, the sensors were separately immersed in anti-CD73 antibody analytes diluted in a gradient with a 0.02% PBST buffer (each antibody was diluted with an initial concentration of 25 nM, resulting in 7 concentration gradients) for the affinity analysis experiment. The times for the ligand-analyte binding and dissociation were 120 s and 300 s, respectively. The data of the antigen-antibody affinity were calculated by regression according to the binding curves using an analysis software. The results are shown in Table 9, indicating that the exemplary antibodies of the present application could cross-recognize the cynomolgus monkey CD73 protein.

**Table 9: Results for the affinity of humanized antibodies to cynomolgus monkey CD73 protein assayed by Fortebio method**

| Test substance | Ka (1/Ms) | Kd (1/s) | KD (M) |
|---|---|---|---|
| Hab7C3C2.5 | 4.08E06 | 2.99E-03 | 7.32E-10 |
| Hab7C3C2.9 | 4.56E06 | 3.88E-03 | 8.52E-10 |
| Hab1F2F9.12 | 2.22E06 | 1.52E-03 | 6.81E-10 |
| Hab1F2F9.15 | 2.09E06 | 1.7E-03 | 8.14E-10 |

### 6.2. Assay on cross-recognition of humanized antibodies with monkey CD73 protein by flow cytometry

In order to characterize the ability of the humanized antibodies prepared in the present application to recognize the cynomolgus monkey CD73 molecule on the cell membrane, CHO-K1 cells capable of stably expressing monkey full-length CD73 were constructed. The cells were seeded in a 96-well plate at a density of 2 × 10⁶ cells/mL, with 100 µL per well. The plate was washed three times with a PBS buffer, and the supernatant was discarded. The antibodies diluted in a gradient with a PBS buffer were added, and the mixture was incubated at 4 °C for 1 h. The cells were washed 3 times with a PBS buffer, followed by labeling with Alexa Fluor 488-labeled goat anti-human IgG antibody at 4 °C in the dark for 50 min. The cells were then washed 3 times with a PBS buffer, and the fluorescence intensity of the cells was read by a flow cytometer.

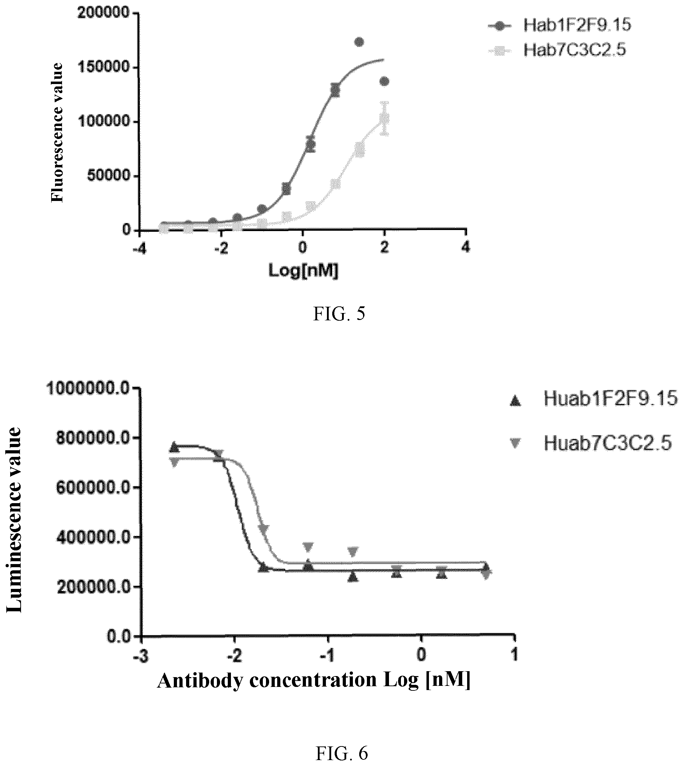

As shown in the results in FIG. 5 and Table 10, the prepared humanized antibodies could bind to the monkey CD73 protein expressed on cells in a dose-dependent manner.

**Table 10. Experimental results for binding of exemplary humanized antibodies of the present application to cynomolgus monkey CD73 on cell surface**

| Test substance | Affinity EC₅₀ at cellular level (nM) |
|---|---|
| Hab7C3C2.5 | 11.34 |
| Hab1F2F9.15 | 1.51 |

### Example 7: Ability of Anti-CD73 Antibodies to Mediate Inhibition of CD73 Enzymatic Activity

### 7.1. Inhibition of CD73 protein enzymatic activity

The recombinant human CD73 protein was diluted to a final concentration of 0.5 µg/mL, and 4-fold gradient dilutions (0-5 nM) of the anti-CD73 antibodies were added, with human IgG1 used as a negative control. The mixture was incubated at 37 °C for 1 h. ATP (75 µM) and AMP (94 µM) were added to each well, and the mixture was incubated at 37 °C for 1 h. An equal volume of CellTiter-Glo containing luciferase was added to each well, and the self-luminescence values were read by a elisa plate reader. The results are shown in FIG. 6 and Table 11, indicating that Hab7C3C.5 and Hab 1F2F9.15 could inhibit the enzymatic activity of free CD73 protein.

**Table 11. Data for inhibitory activity of humanized antibodies on CD73 protein enzymatic activity**

| Test substance | Protein enzymatic activity inhibition IC₅₀ (nM) |
|---|---|
| Hab7C3C2.5 | 0.018 |
| Hab1F2F9.15 | 0.011 |

### 7.2. Inhibitory activity on CD73 enzymatic activity on human MDA-MB-231 cells

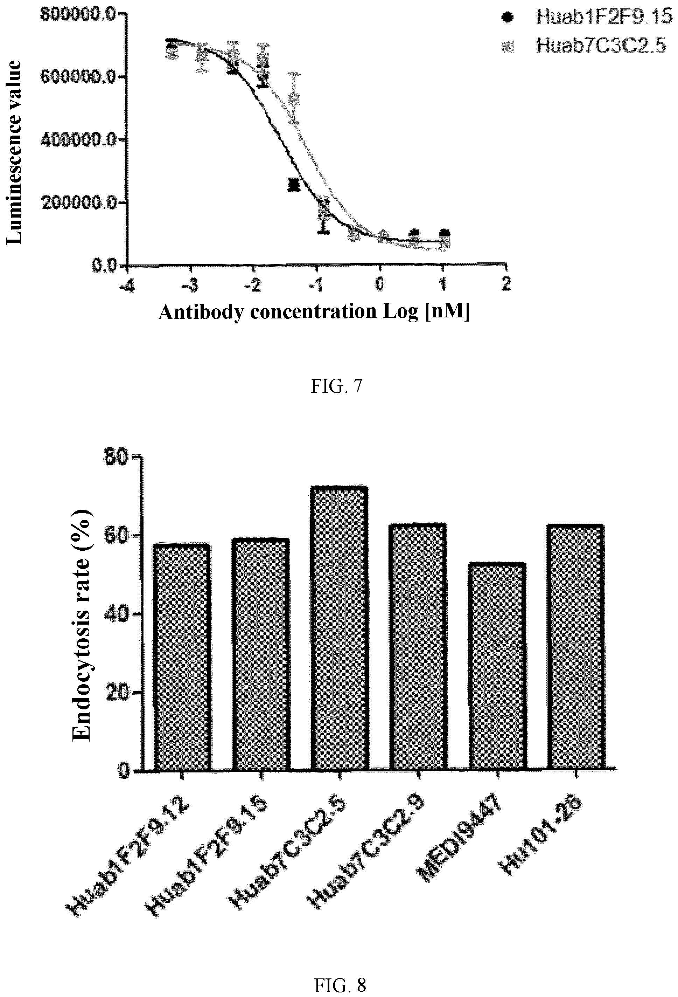

In the presence of different doses of 3-fold gradient dilutions (0-10 nM) of the anti-CD73 antibodies or isotype control antibody (human IgG1) at 50 µL/well, MDA-MB-231 cells expressing CD73 were seeded in a 96-well plate at 2 × 10⁵ cells/mL, with 100 µL per well. The plate was incubated at 37 °C for 1 h, and then AMP (1800 µM) was added to each well at 50 µL/well, followed by incubation at 37 °C for 2 h. The cells were centrifuged at 1000 rpm for 5 min. 50 µL of supernatant was transferred to another 96-well plate, and 50 µL of ATP was then added to achieve a final concentration of 75 µM. CellTiter-Glo reagent (Promega) was added in a 1:1 ratio, and the cellular CD73 enzymatic activity was measured. The results are shown in FIG. 7 and Table 12, indicating that Hab7C3C.5 and Hab1F2F9.15 could inhibit the CD73 enzymatic activity on MDA-MB-231 cells.

**Table 12. Inhibitory Activity of humanized antibodies on CD73 enzymatic activity on MDA-MB-231 cells**

| Test substance | Cellular enzymatic activity inhibition IC₅₀ (nM) |
|---|---|
| Hab7C3C2.5 | 0.027 |
| Hab1F2F9.15 | 0.069 |

### Example 8: Antibody-Induced Internalization of Cell Surface CD73

In this example, the antibody-mediated internalization of CD73 was assessed by flow cytometry. The cells were suspended in a serum-free DMEM medium and incubated on ice for 1 h with each test antibody at a concentration of 10 µg/mL. The cells were washed three times with a serum-free DMEM medium. The cells incubated with different antibodies were separately suspended in a serum-free DMEM medium, and each suspension was divided into two portions, which were then respectively plated into two 24-well plates. One culture plate was placed in an incubator at 37 °C for incubation, and the other plate was placed in a refrigerator at 4 °C as a control group; both were cultured for 24 h. The cells were collected and washed, and then an Alexa Fluor 488-labeled goat anti-human secondary antibody was added. Staining was performed in the dark at 4 °C for 1 h. The cells were then washed 3 times in a PBS buffer and analyzed by a flow cytometer.

The results are shown in FIG. 8, indicating that the exemplary antibodies of the present application could induce the internalization of about 60% of the CD73 receptors, with about 40% of the receptors retaining the initial surface staining. All calculated values represent the percentage of internalization compared to the control (set at 100%).

### Example 9: Identification of Epitopes Recognized by Antibodies

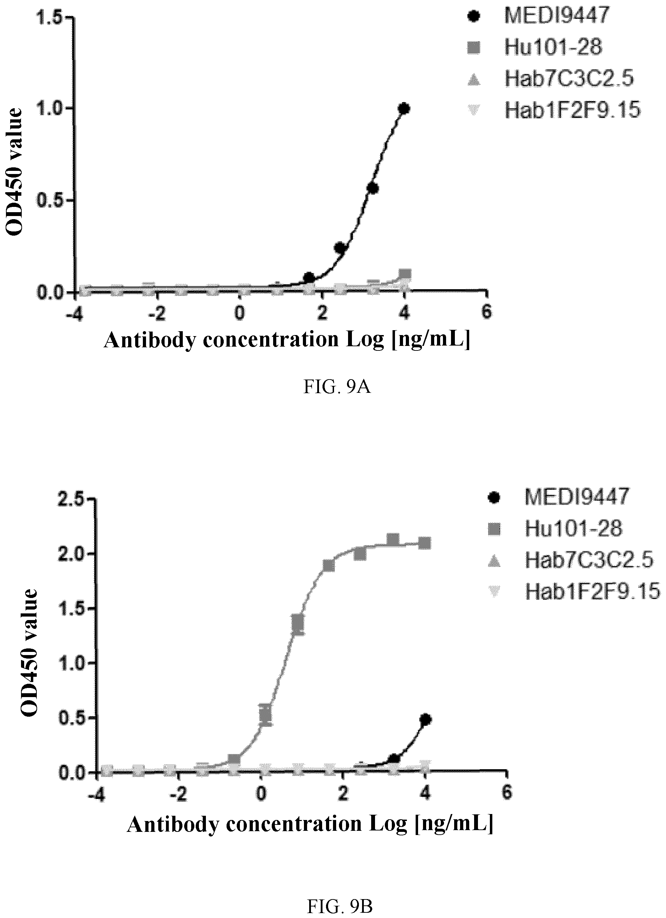

In this example, the C-terminal portion (SEQ ID NO. 41) and the N-terminal portion (SEQ ID NO. 42) of the CD73 protein were separately constructed, and the epitopes recognized by the antibodies were detected by ELISA method. Elisa plates were respectively coated with the CD73 N-terminal and C-terminal proteins at a concentration of 1 µg/mL. The test antibodies were diluted in a gradient. The obtained affinity results are shown in FIGs. 9A (N-terminal) and 9B (C-terminal). The experimental results indicate that the binding epitopes of Hab7C3C.5 and Hab1F2F9.15 were different from those of the control antibodies (MEDI9447 and Hu101-28); Hab7C3C.5 and Hab1F2F9.15 did not bind to the N-terminus and C-terminus of the CD73 protein, while MEDI9447 bound to the N-terminus of the CD73 protein, and Hu101-28 bound to the C-terminus of the CD73 protein. The binding epitopes of Hab7C3C.5 and Hab1F2F9.15 of the present application were significantly different from those of MEDI9447 and Hu101-28.

### Example 10: Evaluation of Biological Activity of Anti-CD73 Antibodies in Animals

### Evaluation of the efficacy of a chimeric antibody alone as a monotherapy in PBMC-immune reconstituted mice

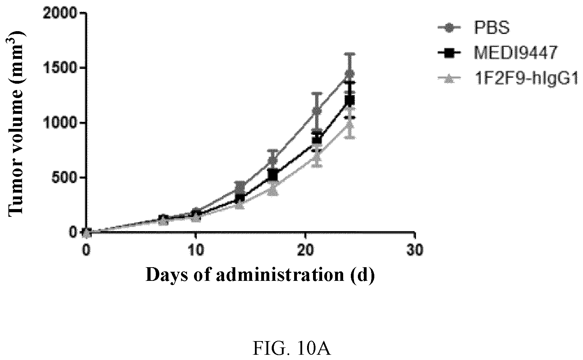

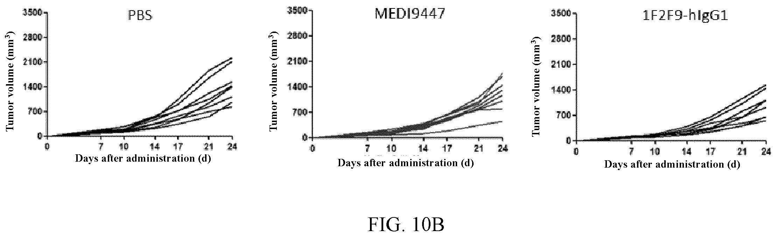

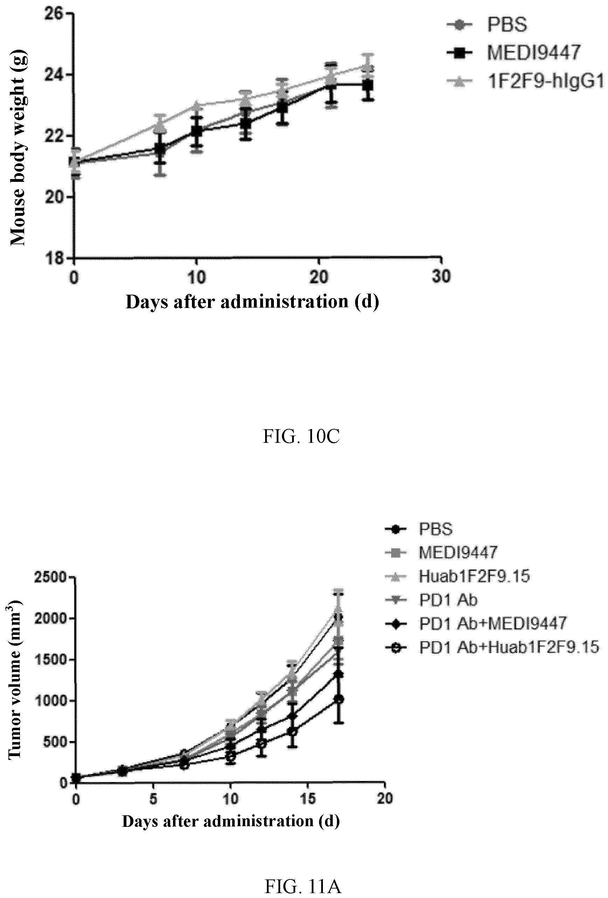

The efficacy of a 1F2F9 chimeric antibody as a monotherapy was evaluated in a tumor xenograft model. A mixture of 4 × 10⁶ A375 melanoma cells and 4 × 10⁵ human PBMCs was mixed with matrigel at a 1:1 ratio, and the resulting mixture was inoculated into NCG immunodeficient mice. On the day of transplantation, the 1F2F9 chimeric antibody or a PBS control was injected intraperitoneally at 30 mg/kg once every other day. On day 24 after the treatment, the tumor size in multiple mice reached the upper limit (2000 mm³). Therefore, day 24 after the treatment was chosen as the data endpoint for analysis. In the control group, the mean tumor volume on day 24 after the treatment was 1448 ± 176.76 mm³ (mean ± standard error). Compared to the control group, administration of 1F2F9-hIgG1 and MEDI9447 reduced tumor growth, resulting in tumor inhibition rates of 31.14% and 16.88%, respectively, with tumor volumes of 997.37 ± 131.39 mm³ and 1203.95 ± 158.01 mm³, respectively (FIG. 10A). The results for individual tumor volumes are shown in FIG. 10B (with multiple lines representing multiple mice, respectively). The body weight changes in each experimental group were not significant (FIG. 10C), indicating that the treatment was well tolerated.

### Example 11: Evaluation of Efficacy of CD73 Humanized Antibodies in Genetically Engineered Mice

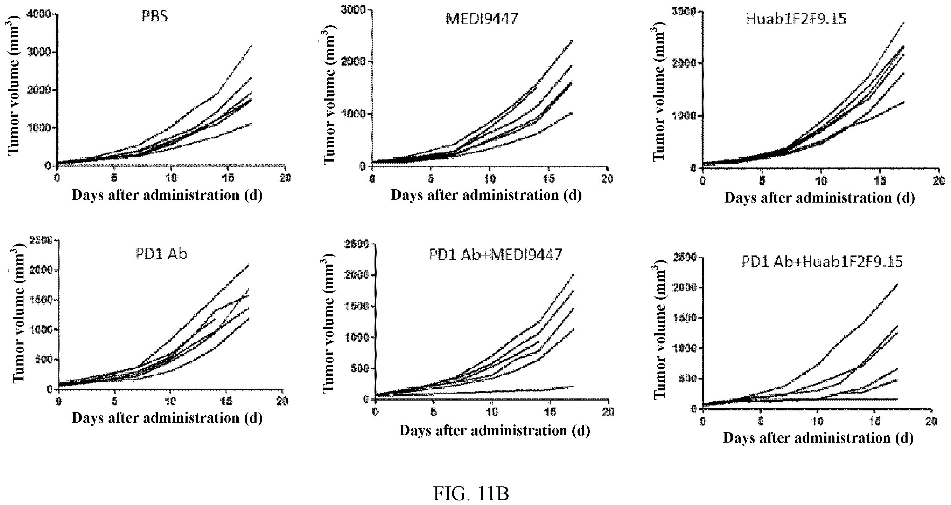

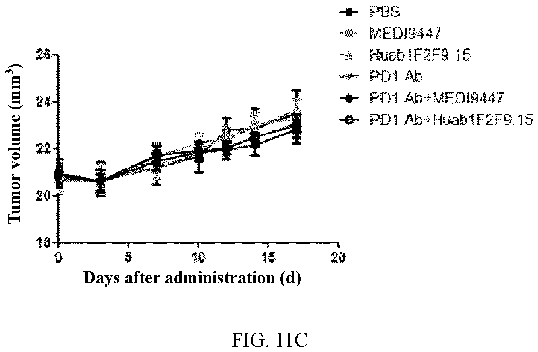

In this example, the efficacy of Huab1F2F9.15, an anti-PD-1 antibody, and a combination thereof was evaluated using human CD73/PD1/PDL1 knock-in BALB/c miceand CT26 cells overexpressing human CD73/PDL1 (a colon cancer cell line). 1 × 10⁶ CT26-hCD73/hPDL1 cells were inoculated subcutaneously into each of the genetically engineered mice. When the mean tumor volume was about 60-80 mm³, the mice were grouped according to the tumor volume. The day of grouping was defined as D0, and administration was started on the day of grouping. Each antibody or the PBS control was injected intraperitoneally at a specified dose twice a week. The mice were treated with the anti-PD-1 antibody alone (1 mg/kg), the anti-CD73 antibody alone (Huab1F2F9.15 or MEDI9447; 15 mg/kg), or a combination of the anti-PD-1 antibody and the anti-CD73 antibody (1 mg/kg + 15 mg/kg). The results indicate that administration of the combination of Huab1F2F9.15 with the anti-PD-1 antibody and the combination of MEDI9447 with the anti-PD1 antibody reduced tumor growth, resulting in tumor inhibition rates of 53.74% and 37.67%, respectively; the effect of the combination of Huab1F2F9.15 with the anti-PD-1 antibody was significantly better than that of the combination of MEDI9447 with the anti-PD1 antibody (FIG. 11A). The results for individual tumor volumes are shown in FIG. 11B (with multiple lines representing different mice, respectively). The body weight changes in each experimental group were not significant (FIG. 11C), indicating that the treatment was well tolerated.

Based on the above experimental results, the present application obtained antibodies with high affinity for recognizing CD73 protein and CD73 at the cellular level. The tested representative antibodies could potently inhibit the enzymatic activity of CD73, and at the same time could induce the internalization of CD73 protein on tumor cells, which further reduced the enzymatic activity of CD73 by reducing the CD73 molecules on the cell surface, thereby achieving the effect of inhibiting the CD73 enzymatic activity through multiple pathways. Epitope analysis shows that the exemplary antibodies of the present application did not bind to either the separate N-terminal and C-terminal portions of the CD73 protein, demonstrating differentiated epitope binding characteristics as compared to antibodies in the prior art such as MEDI9447 and Hu101-28. The animal *in-vivo* experiments confirm that the 1F2F9 chimeric antibody and the humanized Huab1F2F9.15 antibody could effectively inhibit the proliferation of tumor cells in animals. In particular, the humanized Huab1F2F9.15 antibody used in combination with the anti-PD-1 antibody achieved an excellent tumor inhibition effect and showed good tolerability.

The use of any and all examples or exemplary language (e.g., "such as") provided herein is solely intended to better illustrate the present application and is not intended to limit the scope of the present application, unless otherwise specified. The language in the specification should not be construed as indicating any unclaimed elements as essential for the implementation of the present application.

All publications and patent applications cited in this specification are incorporated herein by reference as if each individual publication or patent application was specifically and individually indicated to be incorporated by reference. Moreover, any theories, mechanisms, proofs, or findings described herein are intended to further enhance the understanding of the present application and are not intended to limit the present application in any way to such theories, mechanisms, proofs, or findings. Although the present application has been illustrated and described in detail in the drawings and foregoing description, it should be considered as illustrative rather than restrictive.

## Claims

1. An anti-CD73 antibody or an antigen-binding portion thereof, comprising a heavy chain variable region and a light chain variable region, wherein the heavy chain variable region comprises a heavy chain CDR1 (HCDR1), a heavy chain CDR2 (HCDR2), and a heavy chain CDR3 (HCDR3), and the light chain variable region comprises a light chain CDR1 (LCDR1), a light chain CDR2 (LCDR2), and a light chain CDR3 (LCDR3), wherein
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, the sequence of the HCDR3 is the sequence set forth in SEQ ID NO. 3 or has at least 80%, 85%, 90%, 95% or 99% identity to the sequence set forth in SEQ ID NO. 3, the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, the sequence of the HCDR3 is the sequence set forth in SEQ ID NO. 11 or has at least 80%, 85%, 90%, 95% or 99% identity to the sequence set forth in SEQ ID NO. 11, the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15;
wherein the sequences of the HCDRs and LCDRs are defined according to Kabat.

2. The anti-CD73 antibody or the antigen-binding portion thereof as claimed in claim 1, wherein the heavy chain variable region has the amino acid sequence set forth in SEQ ID NO. 4, 18, 19, 20, 22, 24, 12, 25, 26, 28, 29 or 31, or an amino acid sequence having at least 80%, at least 85%, at least 90%, at least 95% or at least 99% identity to the amino acid sequence set forth in SEQ ID NO. 4, 18, 19, 20, 22, 24, 12, 25, 26, 28, 29 or 31.

3. The anti-CD73 antibody or the antigen-binding portion thereof as claimed in claim 1 or 2, wherein the light chain variable region has the amino acid sequence set forth in SEQ ID NO. 8, 33, 34, 16, 36, 37 or 38, or an amino acid sequence having at least 80%, at least 85%, at least 90%, at least 95% or at least 99% identity to the amino acid sequence set forth in SEQ ID NO. 8, 33, 34, 16, 36, 37 or 38.

4. The anti-CD73 antibody or the antigen-binding portion thereof as claimed in any one of claims 1-3, wherein
the sequence of the heavy chain variable region is the sequence set forth in SEQ ID NO. 4, and the sequence of the light chain variable region is the sequence set forth in SEQ ID NO. 8; or
the sequence of the heavy chain variable region is the sequence set forth in SEQ ID NO. 18, and the sequence of the light chain variable region is the sequence set forth in SEQ ID NO. 33; or
the sequence of the heavy chain variable region is the sequence set forth in SEQ ID NO. 18, and the sequence of the light chain variable region is the sequence set forth in SEQ ID NO. 34; or
the sequence of the heavy chain variable region is the sequence set forth in SEQ ID NO. 19, and the sequence of the light chain variable region is the sequence set forth in SEQ ID NO. 33; or
the sequence of the heavy chain variable region is the sequence set forth in SEQ ID NO. 19, and the sequence of the light chain variable region is the sequence set forth in SEQ ID NO. 34; or
the sequence of the heavy chain variable region is the sequence set forth in SEQ ID NO. 20, and the sequence of the light chain variable region is the sequence set forth in SEQ ID NO. 33; or
the sequence of the heavy chain variable region is the sequence set forth in SEQ ID NO. 20, and the sequence of the light chain variable region is the sequence set forth in SEQ ID NO. 34; or
the sequence of the heavy chain variable region is the sequence set forth in SEQ ID NO. 22, and the sequence of the light chain variable region is the sequence set forth in SEQ ID NO. 33; or
the sequence of the heavy chain variable region is the sequence set forth in SEQ ID NO. 22, and the sequence of the light chain variable region is the sequence set forth in SEQ ID NO. 34; or
the sequence of the heavy chain variable region is the sequence set forth in SEQ ID NO. 24, and the sequence of the light chain variable region is the sequence set forth in SEQ ID NO. 33; or
the sequence of the heavy chain variable region is the sequence set forth in SEQ ID NO. 24, and the sequence of the light chain variable region is the sequence set forth in SEQ ID NO. 34; or
the sequence of the heavy chain variable region is the sequence set forth in SEQ ID NO. 12, and the sequence of the light chain variable region is the sequence set forth in SEQ ID NO. 16; or
the sequence of the heavy chain variable region is the sequence set forth in SEQ ID NO. 25, and the sequence of the light chain variable region is the sequence set forth in SEQ ID NO. 36; or
the sequence of the heavy chain variable region is the sequence set forth in SEQ ID NO. 25, and the sequence of the light chain variable region is the sequence set forth in SEQ ID NO. 37; or
the sequence of the heavy chain variable region is the sequence set forth in SEQ ID NO. 25, and the sequence of the light chain variable region is the sequence set forth in SEQ ID NO. 38; or
the sequence of the heavy chain variable region is the sequence set forth in SEQ ID NO. 26, and the sequence of the light chain variable region is the sequence set forth in SEQ ID NO. 36; or
the sequence of the heavy chain variable region is the sequence set forth in SEQ ID NO. 26, and the sequence of the light chain variable region is the sequence set forth in SEQ ID NO. 37; or
the sequence of the heavy chain variable region is the sequence set forth in SEQ ID NO. 26, and the sequence of the light chain variable region is the sequence set forth in SEQ ID NO. 38; or
the sequence of the heavy chain variable region is the sequence set forth in SEQ ID NO. 28, and the sequence of the light chain variable region is the sequence set forth in SEQ ID NO. 36; or
the sequence of the heavy chain variable region is the sequence set forth in SEQ ID NO. 28, and the sequence of the light chain variable region is the sequence set forth in SEQ ID NO. 37; or
the sequence of the heavy chain variable region is the sequence set forth in SEQ ID NO. 28, and the sequence of the light chain variable region is the sequence set forth in SEQ ID NO. 38; or
the sequence of the heavy chain variable region is the sequence set forth in SEQ ID NO. 29, and the sequence of the light chain variable region is the sequence set forth in SEQ ID NO. 36; or
the sequence of the heavy chain variable region is the sequence set forth in SEQ ID NO. 29, and the sequence of the light chain variable region is the sequence set forth in SEQ ID NO. 37; or
the sequence of the heavy chain variable region is the sequence set forth in SEQ ID NO. 29, and the sequence of the light chain variable region is the sequence set forth in SEQ ID NO. 38; or
the sequence of the heavy chain variable region is the sequence set forth in SEQ ID NO. 31, and the sequence of the light chain variable region is the sequence set forth in SEQ ID NO. 36; or
the sequence of the heavy chain variable region is the sequence set forth in SEQ ID NO. 31, and the sequence of the light chain variable region is the sequence set forth in SEQ ID NO. 37; or
the sequence of the heavy chain variable region is the sequence set forth in SEQ ID NO. 31, and the sequence of the light chain variable region is the sequence set forth in SEQ ID NO. 38.

5. An anti-CD73 antibody or an antigen-binding portion thereof, comprising a heavy chain variable region and a light chain variable region, wherein
the heavy chain variable region has the amino acid sequence set forth in SEQ ID NO. 4, 18, 19, 20, 22, 24, 12, 25, 26, 28, 29 or 31, or an amino acid sequence having at least 80%, at least 85%, at least 90%, at least 95% or at least 99% identity to the amino acid sequence set forth in SEQ ID NO. 4, 18, 19, 20, 22, 24, 12, 25, 26, 28, 29 or 31; and/or
the light chain variable region has the amino acid sequence set forth in SEQ ID NO. 8, 33, 34, 16, 36, 37 or 38, or an amino acid sequence having at least 80%, at least 85%, at least 90%, at least 95% or at least 99% identity to the amino acid sequence set forth in SEQ ID NO. 8, 33, 34, 16, 36, 37 or 38.

6. An anti-CD73 antibody or an antigen-binding portion thereof, comprising a heavy chain variable region, wherein the heavy chain variable region comprises a heavy chain CDR1 (HCDR1), a heavy chain CDR2 (HCDR2), and a heavy chain CDR3 (HCDR3), wherein
(1) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 2 or has at least 80%, 85%, 90%, 95% or 99% identity to the sequence set forth in SEQ ID NO. 2, and the sequence of the HCDR3 is the sequence set forth in SEQ ID NO. 3 or has at least 80%, 85%, 90%, 95% or 99% identity to the sequence set forth in SEQ ID NO. 3; or
(2) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 17 or has at least 80%, 85%, 90%, 95% or 99% identity to the sequence set forth in SEQ ID NO. 17, and the sequence of the HCDR3 is the sequence set forth in SEQ ID NO. 3 or has at least 80%, 85%, 90%, 95% or 99% identity to the sequence set forth in SEQ ID NO. 3; or
(3) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 21 or has at least 80%, 85%, 90%, 95% or 99% identity to the sequence set forth in SEQ ID NO. 21, and the sequence of the HCDR3 is the sequence set forth in SEQ ID NO. 3 or has at least 80%, 85%, 90%, 95% or 99% identity to the sequence set forth in SEQ ID NO. 3; or
(4) the sequence of the HCDR1 is the sequence set forth in SEQ ID NO. 1 or has at least 80%, 85%, 90%, 95% or 99% identity to the sequence set forth in SEQ ID NO. 1, the sequence of the HCDR2 is the sequence set forth in SEQ ID NO. 23 or has at least 80%, 85%, 90%, 95% or 99% identity to the sequence set forth in SEQ ID NO. 23, and the sequence of the HCDR3 is the sequence set forth in SEQ ID NO. 3 or has at least 80%, 85%, 90%, 95% or 99% identity to the sequence set forth in SEQ ID NO. 3; or
(5) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 10 or has at least 80%, 85%, 90%, 95% or 99% identity to the sequence set forth in SEQ ID NO. 10, and the sequence of the HCDR3 is the sequence set forth in SEQ ID NO. 11 or has at least 80%, 85%, 90%, 95% or 99% identity to the sequence set forth in SEQ ID NO. 11; or
(6) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 27 or has at least 80%, 85%, 90%, 95% or 99% identity to the sequence set forth in SEQ ID NO. 27, and the sequence of the HCDR3 is the sequence set forth in SEQ ID NO. 11 or has at least 80%, 85%, 90%, 95% or 99% identity to the sequence set forth in SEQ ID NO. 11; or
(7) the sequence of the HCDR1 is the sequence set forth in SEQ ID NO. 9 or has at least 80%, 85%, 90%, 95% or 99% identity to the sequence set forth in SEQ ID NO. 9, the sequence of the HCDR2 is the sequence set forth in SEQ ID NO. 30 or has at least 80%, 85%, 90%, 95% or 99% identity to the sequence set forth in SEQ ID NO. 30, and the sequence of the HCDR3 is the sequence set forth in SEQ ID NO. 11 or has at least 80%, 85%, 90%, 95% or 99% identity to the sequence set forth in SEQ ID NO. 11;
wherein the sequences of the HCDRs are defined according to Kabat.

7. An anti-CD73 antibody or an antigen-binding portion thereof, comprising a light chain variable region, wherein the light chain variable region comprises a light chain CDR1 (LCDR1), a light chain CDR2 (LCDR2), and a light chain CDR3 (LCDR3), wherein
(1) the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 6 or has at least 80%, 85%, 90%, 95% or 99% identity to the sequence set forth in SEQ ID NO. 6, and the sequence of the LCDR3 is the sequence set forth in SEQ ID NO. 7 or has at least 80%, 85%, 90%, 95% or 99% identity to the sequence set forth in SEQ ID NO. 7; or
(2) the sequence of the LCDR1 is the sequence set forth in SEQ ID NO. 5 or has at least 80%, 85%, 90%, 95% or 99% identity to the sequence set forth in SEQ ID NO. 5, the sequence of the LCDR2 is the sequence set forth in SEQ ID NO. 32 or has at least 80%, 85%, 90%, 95% or 99% identity to the sequence set forth in SEQ ID NO. 32, and the sequence of the LCDR3 is the sequence set forth in SEQ ID NO. 7 or has at least 80%, 85%, 90%, 95% or 99% identity to the sequence set forth in SEQ ID NO. 7; or
(3) the sequence of the LCDR1 is the sequence set forth in SEQ ID NO. 13 or has at least 80%, 85%, 90%, 95% or 99% identity to the sequence set forth in SEQ ID NO. 13, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15; or
(4) the sequence of the LCDR1 is the sequence set forth in SEQ ID NO. 35 or has at least 80%, 85%, 90%, 95% or 99% identity to the sequence set forth in SEQ ID NO. 35, the sequence of the LCDR2 is the sequence set forth in SEQ ID NO. 14 or has at least 80%, 85%, 90%, 95% or 99% identity to the sequence set forth in SEQ ID NO. 14, and the sequence of the LCDR3 is the sequence set forth in SEQ ID NO. 15 or has at least 80%, 85%, 90%, 95% or 99% identity to the sequence set forth in SEQ ID NO. 15;
wherein the sequences of the LCDRs are defined according to Kabat.

8. The antibody or the antigen-binding portion thereof as claimed in any one of claims 1-7, wherein
the anti-CD73 antibody or the antigen-binding portion thereof binds to human CD73; and/or
the anti-CD73 antibody or the antigen-binding portion thereof has ADCC activity; and/or
the anti-CD73 antibody or the antigen-binding portion thereof has CDC activity; and/or
the anti-CD73 antibody is an intact antibody, a single-chain fragment variable (scFv), an scFv-Fc, a bispecific antibody, or a multispecific antibody; and/or
the antigen-binding portion of the anti-CD73 antibody is a Fab, a Fab', an Fv, or an F(ab')₂; and/or
the anti-CD73 antibody is a murine antibody, a chimeric antibody, a humanized antibody, or a fully human antibody; and/or
the anti-CD73 antibody is a monoclonal antibody; and/or
the anti-CD73 antibody is of the IgG1, IgG2 or IgG4 isotype; and/or
the anti-CD73 antibody comprises a light chain constant region of the κ subtype or the λ subtype; and/or
the anti-CD73 antibody comprises a human IgG1 heavy chain constant region and a human κ light chain constant region; and/or
the anti-CD73 antibody comprises a wild-type Fc region; and/or
the anti-CD73 antibody comprises an engineered Fc region that confers reduced ADCC and/or CDC effects to the antibody, for example, the antibody has a human IgG1 heavy chain constant region and comprises a mutation at one or more of positions L234, L235, P329 and P331, such as one or more of mutations L234A, L235A, P329A and P331S, wherein the residues are numbered according to the EU numbering system.

9. The antibody or the antigen-binding portion thereof as claimed in any one of claims 1-8, wherein the antibody has a heavy chain variable region set forth in SEQ ID NO. 20, a light chain variable region set forth in SEQ ID NO. 33, a heavy chain constant region set forth in SEQ ID NO. 39, and a light chain constant region set forth in SEQ ID NO. 40.

10. The antibody or the antigen-binding portion thereof as claimed in any one of claims 1-8, wherein the antibody has a heavy chain variable region set forth in SEQ ID NO. 31, a light chain variable region set forth in SEQ ID NO. 38, a heavy chain constant region set forth in SEQ ID NO. 39, and a light chain constant region set forth in SEQ ID NO. 40.

11. The antibody or the antigen-binding portion thereof as claimed in any one of claims 1-10 for use in a medical therapy.

12. An isolated nucleic acid molecule encoding the anti-CD73 antibody or the antigen-binding portion thereof as claimed in any one of claims 1-11.

13. A vector, comprising the nucleic acid molecule as claimed in claim 12.

14. A host cell, comprising the nucleic acid molecule as claimed in claim 12 or the vector as claimed in claim 13.

15. An antibody-drug conjugate, comprising the anti-CD73 antibody or the antigen-binding portion thereof as claimed in any one of claims 1-11 conjugated to a therapeutic agent.

16. The antibody-drug conjugate as claimed in claim 15, wherein the therapeutic agent is an anti-tumor drug, such as a cytotoxic drug, an
immunopotentiator, or a radioisotope.

17. A pharmaceutical composition, comprising the anti-CD73 antibody or the antigen-binding portion thereof as claimed in any one of claims 1-11 or the antibody-drug conjugate as claimed in claim 15 or 16, and a pharmaceutically acceptable carrier.

18. The pharmaceutical composition as claimed in claim 17 for use in the treatment of a tumor, particularly a tumor expressing CD73 (CD73+) on the surface of tumor cells, in an individual.

19. The pharmaceutical composition as claimed in claim 18, wherein the tumor is a solid tumor.

20. The pharmaceutical composition as claimed in claim 18, wherein the tumor is a malignant tumor.

21. The pharmaceutical composition as claimed in claim 18, wherein the tumor is a cancer.

22. The pharmaceutical composition as claimed in claim 18, wherein the tumor is selected from: ovarian cancer, breast cancer, melanoma, colorectal cancer, gastric cancer, renal cancer, prostate cancer, pancreatic cancer, esophageal cancer, head and neck cancer, thyroid cancer, lung cancer, bladder cancer, cervical cancer, colon cancer, liver cancer, peritoneal cancer, hepatocellular carcinoma, glioblastoma, urinary tract cancer, rectal cancer, endometrial cancer, uterine cancer, salivary gland cancer, squamous cell carcinoma, vulval cancer, anal cancer, penile cancer, brain cancer, lymphoma (e.g., B-cell lymphoma), leukemia, and metastases of the tumors described above.

23. The pharmaceutical composition as claimed in any one of claims 18-22 for use in combination with an immune checkpoint inhibitor, an A2AR antagonist, or a STAT-3 inhibitor for the treatment of the tumor.

24. The pharmaceutical composition as claimed in claim 23, wherein the immune checkpoint inhibitor is an anti-PD-1 antibody, an anti-PD-L1 antibody, or an anti-CTLA4 antibody.

25. The pharmaceutical composition as claimed in claim 23, wherein the immune checkpoint inhibitor is an anti-PD-1 antibody.

26. Use of the anti-CD73 antibody or the antigen-binding portion thereof as claimed in any one of claims 1-11, the nucleic acid molecule as claimed in claim 12, the vector as claimed in claim 13, the host cell as claimed in claim 14, or the antibody-drug conjugate as claimed in claim 15 or 16 for the manufacturing of a medicament for the treatment of a tumor, particularly a tumor expressing CD73 (CD73+) on the surface of tumor cells, in an individual.

27. The use as claimed in claim 26, wherein the tumor is a solid tumor.

28. The use as claimed in claim 26, wherein the tumor is a malignant tumor.

29. The use as claimed in claim 26, wherein the tumor is a cancer.

30. The use as claimed in claim 26, wherein the tumor is selected from: ovarian cancer, breast cancer, melanoma, colorectal cancer, gastric cancer, renal cancer, prostate cancer, pancreatic cancer, esophageal cancer, head and neck cancer, thyroid cancer, lung cancer, bladder cancer, cervical cancer, colon cancer, liver cancer, peritoneal cancer, hepatocellular carcinoma, glioblastoma, urinary tract cancer, rectal cancer, endometrial cancer, uterine cancer, salivary gland cancer, squamous cell carcinoma, vulval cancer, anal cancer, penile cancer, brain cancer, lymphoma (e.g., B-cell lymphoma), leukemia, and metastases of the tumors described above.

31. The use as claimed in any one of claims 26-30, wherein the medicament is for use in combination with an immune checkpoint inhibitor, an A2AR antagonist, or a STAT-3 inhibitor for the treatment of the tumor.

32. The use as claimed in claim 31, wherein the immune checkpoint inhibitor is an anti-PD-1 antibody, an anti-PD-L1 antibody, or an anti-CTLA4 antibody.

33. The use as claimed in claim 31, wherein the immune checkpoint inhibitor is an anti-PD-1 antibody.

34. A method for treating a tumor, particularly a tumor expressing CD73 (CD73+) on the surface of tumor cells, in an individual, wherein the method comprises administering to the individual a therapeutically effective amount of the anti-CD73 antibody or the antigen-binding portion thereof as claimed in any one of claims 1-11, the antibody-drug conjugate as claimed in claim 15 or 16, or the pharmaceutical composition as claimed in claim 17.

35. The method as claimed in claim 34, wherein the tumor is a solid tumor.

36. The method as claimed in claim 34, wherein the tumor is a malignant tumor.

37. The method as claimed in claim 34, wherein the tumor is a cancer.

38. The method as claimed in claim 34, wherein the tumor is selected from: ovarian cancer, breast cancer, melanoma, colorectal cancer, gastric cancer, renal cancer, prostate cancer, pancreatic cancer, esophageal cancer, head and neck cancer, thyroid cancer, lung cancer, bladder cancer, cervical cancer, colon cancer, liver cancer, peritoneal cancer, hepatocellular carcinoma, glioblastoma, urinary tract cancer, rectal cancer, endometrial cancer, uterine cancer, salivary gland cancer, squamous cell carcinoma, vulval cancer, anal cancer, penile cancer, brain cancer, lymphoma (e.g., B-cell lymphoma), leukemia, and metastases of the tumors described above.

39. The method as claimed in any one of claims 34-38, wherein the method further comprises administering the anti-CD73 antibody or the antigen-binding portion thereof, the antibody-drug conjugate, or the pharmaceutical composition in combination with an immune checkpoint inhibitor, an A2AR antagonist, or a STAT-3 inhibitor to the individual, so as to treat the tumor.

40. The method as claimed in claim 39, wherein the immune checkpoint inhibitor is an anti-PD-1 antibody, an anti-PD-L1 antibody, or an anti-CTLA4 antibody.

41. The method as claimed in claim 39, wherein the immune checkpoint inhibitor is an anti-PD-1 antibody.

42. A conjugate, comprising the anti-CD73 antibody or the antigen-binding portion thereof as claimed in any one of claims 1-11 and a detectable label.

43. A fusion protein, comprising the anti-CD73 antibody or the antigen-binding portion thereof as claimed in any one of claims 1-11.
